(19) Europäisches Patentamt
European Patent Office
Office européen des brevets

(11) EP 1 023 299 B1

(12) EUROPÄISCHE PATENTSCHRIFT

(45) Veröffentlichungstag und Bekanntmachung des Hinweises auf die Patenterteilung:
**27.03.2002 Patentblatt 2002/13**

(21) Anmeldenummer: **98947555.3**

(22) Anmeldetag: **29.09.1998**

(51) Int Cl.[7]: **C07F 9/32**, A61K 31/66

(86) Internationale Anmeldenummer:
**PCT/EP98/06162**

(87) Internationale Veröffentlichungsnummer:
**WO 99/19332 (22.04.1999 Gazette 1999/16)**

# (54) KOMBINATORISCHE ERZEUGUNG VON PHOSPHINSÄUREDERIVATEN

COMBINED GENERATION OF PHOSPHINIC ACID DERIVATIVES

PRODUCTION COMBINEE DE DERIVES D'ACIDE PHOSPHINIQUE

(84) Benannte Vertragsstaaten:
**AT BE CH DE DK ES FR GB IT LI NL**

(30) Priorität: **09.10.1997 US 61619 P**
**10.10.1997 DE 19745628**

(43) Veröffentlichungstag der Anmeldung:
**02.08.2000 Patentblatt 2000/31**

(73) Patentinhaber: **Aventis CropScience GmbH**
**65929 Frankfurt am Main (DE)**

(72) Erfinder:
- **HAAF, Klaus**
  **D-65779 Kelkheim (DE)**
- **PATEK, Marcel**
  **Tucson, AZ 85737 (US)**

(56) Entgegenhaltungen:
**DE-A- 2 346 657**

- **FRUCHTEL J S ET AL: "ORGANIC CHEMISTRY ON SOLID SUPPORTS" ANGEWANDTE CHEMIE. INTERNATIONAL EDITION, Bd. 35, Nr. 1, 19. Januar 1996, Seiten 17-42, XP000548938 in der Anmeldung erwähnt**
- **BOYD E A ET AL: "Multiple Solid Phase Synthesis of (RS)-1-Aminophosphinic Acids" TETRAHEDRON LETTERS, Bd. 37, Nr. 10, 4. März 1996, Seite 1647-1650 XP004030016 in der Anmeldung erwähnt**
- **CAO X ET AL: "COMBINATORIAL METHOD FOR THE SYNTHESIS OF ALPHA-HYDROXY PHOSPHONATES ON WANG RESIN" TETRAHEDRON LETTERS, Bd. 37, Nr. 34, 19. August 1996, Seiten 6073-6076, XP000598590**
- **DORFF P H ET AL: "Solid Phase Synthesis of Phosphinopeptoids as Transition State Analog Inhibitors" TETRAHEDRON LETTERS, Bd. 39, Nr. 21, 21. Mai 1998, Seite 3375-3378 XP004117579**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 023 299 B1

Printed by Jouve, 75001 PARIS (FR)

## Beschreibung

Die Erfindung betrifft das technische Gebiet der Synthese von chemischen Verbindungen mit bestimmten gemeinsamen Strukturmerkmalen, insbesondere auf dem Gebiet der Zwischenprodukte und Wirkstoffe aus der Gruppe der Phosphonigsäuren und Phosphinsäuren und deren Ester.

Phosphorhaltige Verbindungen sind im Stoffwechsel von tierischen und pflanzlichen Organismen häufig anzutreffen. Anhand allgemein bekannter Beispiele ist schon gezeigt worden, daß strukturelle Varianten solcher Verbindungen Wirkstoffe auf dem Gebiet der Pharmazeutika oder Pflanzenschutzmittel sein können. Allerdings besteht ein wachsendes Problem aus der Vielzahl der strukturellen Varianten potentieller Wirkstoffe solche mit gewünschten Eigenschaften herauszufinden. Steigende Anforderungen an die Eigenschaften neuer biologisch aktiver Stoffe für den Pflanzenschutz oder die Medizin haben es mit sich gebracht, daß die Entwicklung eines marktreifen Wirkstoffs mit der Herstellung und Prüfung einer immer größeren Zahlen an Testsubstanzen verbunden ist. Nach Einschätzung vieler Fachleute wird diese Tendenz trotz verfeinerter Kenntnisse über die Biochemie bekannter Wirkstoffe und computerunterstützter Berechnungen von Molekülstrukturen und -eigenschaften ("molecular modelling") anhalten. Um Aufwand an Kosten und Zeit nicht gleichermaßen ansteigen zu lassen, stellt sich für die Forschung nach neuen Wirkstoffen die Aufgabe, effektivere Methoden zur Herstellung großer Zahlen neuartiger oder systematisch variierter Testverbindungen zu entwickeln.

Die Methoden zur systematischen Herstellung großer Zahlen an Testverbindungen und speziell dazu geeigneter Methoden zu deren Analyse und biologischen Prüfung werden unter dem Begriff "kombinatorische Chemie" zusammengefaßt; vgl. z. B. J. S. Früchtel, G. Jung in Angew. Chem. 108 (1996) 1946 oder Angew. Chem. Int. Ed. Engl. 35 (1996) S. 17-42.

Einige kombinatorische Synthesemethoden zielen darauf ab, in möglichst effektiver, standardisierter Weise eine große Zahl von strukturvarianten Verbindungen in möglichst wenigen Reaktionsschritten gemeinsam ("in einem Pool") herzustellen und gemeinsam auf biologische Wirkung zu testen; vgl. z. B. die Divide-Couple-and-Recombine-Methode nach a) K. S. Lam, S. E. Salmon, E. M. Hersh, V. J. Hruby, W. M. Kazmeiersky, R. J. Knapp in Nature 82 (1991) 354, b) A. Furka, F. Sebestyen, M. Asgedom, G. Dibo in Int. J. Pept. Protein Res. 37 (1991) 487. Falls dann eine ganze Gruppe von Verbindungen ("Pool") keine wirksame Verbindung enthält, so reicht im Prinzip ein einziger gemeinsamer Test aus, um diese Strukturvarianten auszuschließen. Falls der gemeinsame Test jedoch Wirksamkeit anzeigt, kann die Variation bei der Herstellung der Testverbindungen gezielt verringert werden, um die Gruppe mit dem Wirkstoff oder den Wirkstoffen einzugrenzen und schließlich die Struktur der wirksamen Verbindungen zu bestimmen. Die beschriebene "Pooling"-Methode läßt sich in der Regel jedoch nicht mehr sinnvoll anwenden, wenn es um die Optimierung von Wirkstoffstrukturen geht und in der Gruppe der Testverbindungen viele ähnlich wirksame Verbindungen vorhanden bzw. zu erwarten sind oder auch wenn größere Mengen der Verbindungen für die Ersttests benötigt werden.

Zur Lösung der letztgenannten Aufgabe geht man oftmals von einer Verbindung mit bekannter biologischer Wirkung aus, der sogenannten Leitstruktur oder Leitverbindung, und variiert die Struktur der Leitverbindung systematisch mit Hilfe eines weitgehend standardisierten ("kombinatorischen) Herstellungsverfahrens durch Einsatz einer großen Zahl unterschiedlicher Edukte. Die jeweils hergestellten Einzelverbindungen werden dann einzeln auf ihre biologische Wirkung getestet, um die Verbindung mit optimaler Wirkung gleicher Wirkungsart herauszufinden.

Zu den bekannten Synthesemethoden aus der kombinatorischen Chemie (siehe J. S. Früchtel, G. Jung in Angew. Chem. 108 (1996) 19-46 und dort zitierter Literatur) gehört eine Gruppe von Methoden, bei welchen der jeweilige Wirkstoff schrittweise festkörpergebunden, vorzugsweise gebunden an ein organisches Polymer (im folgenden als "synthetisches oder natürliches Harz", "Harz" oder "Harzpolymer" bezeichnet), hergestellt wird.

Mit Hilfe der Bindung an den Festkörper, z. B. an das Harz in Form von Teilchen hoher Korngröße oder Kügelchen, werden die Zwischenstufen im Prinzip makroskopisch handhabbar. Die Synthese eines Wirkstoffes über mehrere Reaktionsstufen benötigt dann weniger Aufwand bei der Isolierung und Reinigung als bei konventionellen Methoden, weil diese Schritte in der Regel in Form einer einfachen Filtration und Abspülung der Harzkörper zu bewerkstelligen sind. Das harzgebundene fertige Wirkstoffmolekül muß schließlich noch vom Harz abgespalten werden.

Für die Wahl geeigneter Harz-Molekül-Systeme ergeben sich grundsätzlich Probleme durch den Zielkonflikt, sowohl eine gewünschte hohe Stabilität der Bindung zwischen synthetisierten Molekülteilen und Harz bei der Anwendung unterschiedlicher Reaktionstypen und -bedingungen zu gewährleisten als auch eine schonende Methode zur überwiegenden oder vollständigen Abspaltung des fertigen Syntheseprodukts vom Harz zu ermöglichen.

Der Erfindung liegt die Aufgabe zugrunde, eine kombinatorische Synthesemethode auf Basis harzgebundener Synthesebausteine und -produkte bereitzustellen, welche die Synthese einer breiten Variation von potentiell biologisch aktiven Verbindungen mit einem Phosphoratom und ähnlicher Partialstruktur erlaubt.

Gegenstand der Erfindung ist ein Verfahren unter Verwendung von Zwischenprodukten, die an ein Harzpolymer gebunden sind, zur Herstellung von chemischen Verbindungen der Formel (I),

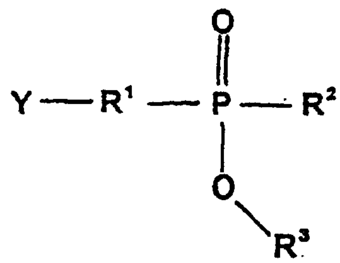

(I)

worin

$R^1$ einen unsubstituierten oder substituierten aromatischen oder heteroaromatischen Rest bedeutet,
$R^2$ Wasserstoff oder einen organischen Rest bedeutet, der auch über Heteroatome mit dem restlichen Teil der Verbindung der Formel (I) verbunden sein kann,
$R^3$ Wasserstoff oder einen über ein C-Atom gebundenen organischen Rest bedeutet und
Y die funktionelle Gruppe bedeutet, die nach Abspaltung der Verbindung (I) vom Harzpolymer am Molekül der Formel (I) gebildet wird,

dadurch gekennzeichnet, daß man

a) ein Harz-Linker-Addukt der Formel (II)

$$[\ \text{Harzpolymer}\ ] - [\ \text{Linker} - Z - E^1 - S^1\ ]_n \quad \text{(II)}$$

worin

[ Harzpolymer ] für den Rest eines Harzes steht, der in der Harz-Linker-Verbindung (II) über n Bindungsstellen mit den n Gruppen der Formel [ Linker-Z-$E^1$-$S^1$] verbunden ist,
Linker jeweils einen organischen Linker bedeutet,
Z eine für den jeweiligen Linker spezifische funktionelle Gruppe oder Bindung bedeutet, aus der nach Abspaltung der Verbindung (I) vom Harzpolymer-Linker-Rest die Gruppe Y in Formel (I) entsteht,
$E^1$ wie $R^1$ in Formel (I) definiert ist oder einen für die Herstellung von $R^1$ in Verbindung (I) geeigneten Rest bedeutet,
$S^1$ eine funktionelle Gruppe ist, die für palladiumkatalysierte Substitutionen analog der Heck-Reaktion geeignet ist,
n die Anzahl der funktionellen Gruppen [Linker-Z-$E^1$-$S^1$] am Harz bedeutet, die vom Molekulargewicht des Harzpolymers abhängt und größer oder gleich 1 ist,

in Gegenwart eines geeigneten Palladiumkatalysators mit einer Verbindung aus der Gruppe der Phosphinate (Derivate der unterphosphorigen Säure) der Formel (III)

$$A^1\text{-O-(PHO)}A^* \quad \text{(III)}$$

worin

$A^1$ Wasserstoff oder einen organischen Rest, vorzugsweise den Rest der Formel $R^3$ in Formel (I) oder einen davon verschiedenen Alkylrest, insbesondere ($C_1$-$C_4$)Alkyl, oder einen Trialkylsilylrest wie Trimethylsilyl (TMS) bedeutet und
A* eine hydrolytisch abtrennbare oder nach einer Zwischenreaktion abtrennbare Gruppe ist, beispielsweise eine Alkylgruppe, ein Trialkylsilylrest, wie TMS, oder Dialkoxymethyl,

vorzugsweise mit einer Verbindung der Formel (III-1), (III-2) oder (III-3),

$$H_2P(=O) - O - A^1 \quad \text{(III-1)}$$

$$A^1 - O - PH - O - A^* \quad \text{(III-2)}$$

$$A^1 - O - P(H)(=O)(A^*) \quad \text{(III-3)}$$

worin $A^1$ und $A^*$ wie oben definiert ist,
insbesondere mit einem Phosphinatderivat der Formel

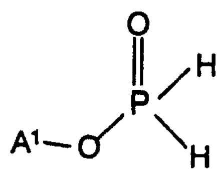
,
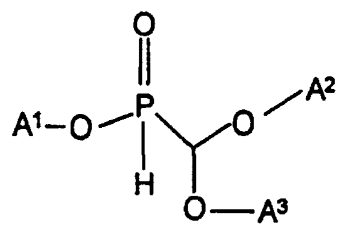

oder

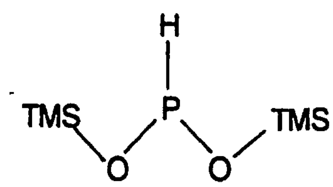

worin $A^1$ einen organischen Rest, vorzugsweise den Rest der Formel $R^3$ in Formel (I) oder einen davon verschiedenen Alkylrest, insbesondere $(C_1-C_4)$Alkyl bedeutet, $A^2$ und $A^3$ jeweils einen Alkylrest wie $(C_1-C_4)$Alkyl bedeuten und TMS = Trimethylsilyl ist,
unter Substitution der Gruppe $S^1$ zu einer harzgebundenen Verbindung der Formel (IV) umsetzt,

$$[\text{ Harzpolymer }]\text{-} [\text{ Linker - Z - } E^1 \text{ - P(H)(=O) - O - } A^1 ]_n \quad \text{(IV)}$$

worin $A^1$ wie in Formel (III) definiert ist, und

b) gegebenenfalls - vorzugsweise im Falle, daß $E^1$ in der in a) eingesetzten Verbindung (II) nicht gleich $R^1$ ist - die Verbindung (IV) in einem oder mehreren weiteren Reaktionsschritten am organischen Rest $E^1$ zum Rest $(E^1)$' derivatisiert und damit eine bzw. mehrere harzgebundene Zwischenstufen der Formel (IV)' erhält,

$$[\text{ Harzpolymer }] - [\text{ Linker - Z - } (E^1)' \text{ - P(H)(=O) - O - } A^1 ]_n \quad \text{(IV)'}$$

worin $A^1$ wie in Formel (III) definiert ist, und

c) gegebenenfalls die Verbindung der Formel (IV) bzw. (IV)' aus Stufe a) bzw. b) zu einer für die harzgebundene Synthese geeigneten, d. h. vor allem unter Berücksichtigung der Reaktivität der Linker-Verknüpfung und der Quellfähigkeit des jeweils eingesetzten Harzes geeigneten Verbindung (V), bzw. (V)' hydrolysiert,

$$[\text{ Harzpolymer }]\text{- }[\text{ Linker - Z - }E^1\text{ - P(H)(=O) - OH }]_n \qquad (V)$$

$$[\text{ Harzpolymer }]\text{- }[\text{ Linker - Z - }(E^1)'\text{ - P(H)(=O) - OH }]_n \qquad (V)'$$

und

d) gegebenenfalls die nach c) erhaltene Verbindung (V) bzw. (V)' zur Verbindung der Formel (VI) bzw. (VI)' verestert,

$$[\text{ Harzpolymer }]\text{ - }[\text{ Linker - Z - }E^1\text{ - P(H)(=O) - O - }R^3\text{ }]_n \qquad (VI)$$

$$[\text{ Harzpolymer }]\text{ - }[\text{ Linker - Z - }(E^1)'\text{ - P(H)(=O) - O - }R^3\text{ }]_n \qquad (VI)'$$

worin

$R^3$ wie $R^3$ in Formel (I) definiert, aber nicht gleich Wasserstoff ist, und

e) gegebenenfalls eine nach a), b), c) oder d) erhaltene Verbindung (IV), (V) oder (VI) bzw. (IV)', (V)' bzw. (VI)', deren gemeinsames Strukturmerkmal die Phosphonigsäure- oder Phosphonigsäureester-gruppe ist, unter Ausbildung einer Phosphor-Kohlenstoffbindung zu Verbindungen der allgemeinen Formeln (VII) oder (VIII) bzw. (VII)' bzw. (VIII)' umsetzt,

$$[\text{ Harzpolymer }]\text{ - }[\text{ Linker - Z - }E^1\text{ - P(}R^2\text{)(=O) - O - }A^4\text{ }]_n \qquad (VII)$$

$$[\text{ Harzpolymer }]\text{- }[\text{ Linker - Z - }(E^1)'\text{ - P(}R^2\text{)(=O) - O - }A^4\text{ }]_n \qquad (VII)'$$

$$[\text{ Harzpolymer }]\text{ - }[\text{ Linker - Z - }E^1\text{ - P(}E^2\text{)(=O) - O - }A^4\text{ }]_n \qquad (VIII)$$

$$[\text{ Harzpolymer }]\text{ - }[\text{ Linker - Z - }(E^1)'\text{ - P(}E^2\text{)(=O) - O - }A^4\text{ }]_n \qquad (VIII)'$$

worin

$R^2$ die in Formel (I) festgelegte Bedeutung hat,
$E^2$ einen organischen Rest bedeutet, der zu dem Rest $R^2$ derivatisiert werden kann,
$A^4$ = $A^1$, H bzw. $R^3$ bedeutet, und

f) die nach vorstehenden Stufen erhaltenen Verbindungen, falls erforderlich, an den Resten $E^1$, $(E^1)'$, $E^2$ und $A^4$ so modifiziert, daß die harzgebundene Verbindung der Formel (IX) erhalten wird,

$$[\text{ Harzpolymer }]\text{ - }[\text{ Linker - Z - }R^1\text{ - P(}R^2\text{)(=O) - O - }R^3\text{ }]_n \qquad (IX)$$

worin $R^1$, $R^2$, $R^3$ wie in Formel (I) definiert sind, und

g) die Verbindung der Formel (I), aus dem Harz-Linker Addukt der Formel (IX) abspaltet,

wobei in den Formeln (IV) bis (IX) und (IV)' bis (VIII)' die Reste [Harzpolymer], Linker, Z wie in Formel (II) definiert sind und $E^1$ bzw. $(E^1)'$ in den Formeln (V) bis (VIII) bzw. (V)' bis (VIII)' wie in Formel (IV) bzw. (IV)' definiert sind.

Gegenstand der Erfindung sind auch die Einzelschritte des erfindungsgemäßen Verfahrens und die neuartigen Verbindungen der Formel (I), (II) und (IV) bis (IX) und (IV)' bis (VIII)'.

Ein besonderer Aspekt der Erfindung liegt in der strukturellen Variationsbreite der herstellbaren Verbindungen der Formel (I). Sie wird vor allem dadurch ermöglicht, daß zahlreiche Derivatisierungsreaktionen an den in Stufe a)

eingeführten Phosphonigsäure- oder Phosphonigsäureestergruppen in Frage kommen, die mit hohen Ausbeuten in den einzelnen Stufen durchgeführt werden können. Besonders überraschend gelingt bereits die Einführung der phosphorhaltigen Gruppen am Harzgerüst mit guter Ausbeute. Denn erfahrungsgemäß gelingen viele der aus der Chemie in freier Lösung bekannten Reaktionen nicht unter analogen Bedingungen, nicht mit guten Ausbeuten oder überhaupt nicht, wenn eine der Reaktionskomponenten an Trägern fixiert worden ist. Die Einführung der Phosphonigsäure- oder Phosphonigsäureestergruppen unter den Bedingungen der palladiumkatalysierten Heck-Reaktion war in Festphasensynthesen bisher nicht bekannt.

Überraschender ist auch die gute Ausbeute und Reinheit der Endprodukte (I), die aufgrund ihrer funktionellen Gruppen sehr polare Moleküle darstellen. Dagegen sind entsprechende Synthesen in freier Lösung teilweise mit extremen Ausbeuteverlusten verknüpft.

In der Formel (I) und den anderen allgemeinen Formeln (II) bis (VII) bedeutet ein organischer Rest einen kohlenstoffhaltigen Rest, beispielsweise einen gegebenenfalls substituierten (hetero)aromatischen Rest oder einen aliphatischen, d.h. nicht aromatischen organischen Rest, der außer C-Atomen und Wasserstoffatomen auch Heteroatome enthalten kann, oder einen entsprechenden araliphatischen Rest.

Die geeigneten organischen Reste können in der Größe recht unterschiedlich sein; vorzugsweise enthält ein organischer Rest inklusive eventuell enthaltender Substituenten weniger als 30 C-Atome, insbesondere 1 bis 20 C-Atome, wobei kleinere Reste mit 1 bis 12 C-Atome in der Regel bevorzugt sind. Als Substituenten eines organischen Restes kommen ebenfalls (hetero)aromatische und aliphatische Reste, inklusive funktionelle Gruppen in Frage, wobei die funktionellen Gruppen vorzugsweise gut mit dem sonst im fixierten Molekülteil vorhandenen funktionellen Gruppen kompatibel sind. Beispielsweise sollten als funktionelle Gruppen keine oxidativen Gruppen vorhanden sein, wenn der Linker oxidationsempfindlich ist und damit schon unter den Bedingungen der kombinatorische Synthese reagieren würde.

Organische Reste sind beispielsweise gegebenenfalls substituierte Kohlenwasserstoffreste oder Kohlenwasserstoffoxyreste. Ein Kohlenwasserstoffrest ist ein geradkettiger, verzweigter oder cyclischer und gesättigter oder ungesättigter oder aromatischer Kohlenwasserstoffrest; z.B. Alkyl, Alkinyl, Cycloalkyl, Cycloalkenyl oder Aryl; vorzugsweise bedeutet ein Kohlenwasserstoffrest Alkyl, Alkenyl oder Alkinyl mit bis zu 12 C-Atomen oder Cycloalkyl mit 3, 4, 5, 6, 7 oder 8 Ringatomen oder Aryl; entsprechendes gilt für einen Kohlenwasserstoffrest in einem Kohlenwasserstoffoxyrest. Organische Reste, die über ein Heteroatom gebunden sein können, schließen auch Gruppen wie Trialkylsilyl wie Trimethylsilyl (TMS) ein.

Aryl bedeutet ein mono-, bi- oder polycyclisches, carbocyclisches aromatisches Ringsystem; im substituierten Fall, oder genauer im cyclisch substituierten Fall, werden insbesondere auch bicyclische oder polycyclische Ringsysteme mit mindestes einem aromatischen Ring, der mit einem oder mehreren cycloaliphatischen, gegebenenfalls teilungesättigten Ringen anelliert ist, eingeschlossen. Gegebenfalls cyclisch substituiertes Aryl ist beispielsweise Phenyl, Naphthyl, Tetrahydronaphthyl, Indenyl, Indanyl, Pentalenyl, Fluorenyl und ähnliches, wobei die genannten Ringsysteme im allgemein substituierten Fall noch weiter substituiert sein können; vorzugsweise ist Aryl ein unsubstituierter Phenyl-oder Naphthylring; substituiertes Aryl ist vorzugsweise ein Phenylrest, der unsubstituiert oder substituiert ist, wobei die Substituenten keinen ankondensierten Ring bedeuten.

Heteroaryl oder ein heteroaromatischer Rest bedeutet ein mono-, bi- oder polycyclisches aromatisches Ringsystem, in dem mindestens 1 Ring ein oder mehrere Heteroatome enthält, beispielsweise Pyridyl, Pyrimidinyl, Pyridazinyl, Pyrazinyl, Triazinyl, Thienyl, Thiazolyl, Oxazolyl, Isoxazolyl, Furyl, Pyrrolyl, Pyrazolyl und Imidazolyl. Im substituierten Fall werden insbesondere auch bicyclische oder polycyclische aromatische, benzokondensierte oder mit cycloaliphatischen Ringen anellierte Verbindungen, z.B. Chinolinyl, Benzoxazolyl etc. eingeschlossen. Heteroaryl schließt auch einen heteroaromatischen Ring ein, der vorzugsweise 5- oder 6-gliedrig ist und 1,2- oder 3 Heteroringatome, insbesondere aus der Gruppe N, O und S enthält.

Ein heterocyclischer Rest (Heterocyclyl) oder Ring (Heterocyclus) kann gesättigt, ungesättigt oder heteroaromatisch (Heteroaryl) sein; er enthält ein oder mehrere Heteroringatome, vorzugsweise aus der Gruppe N, O und S; vorzugsweise ist er ein nicht aromatischer Ring mit 3 bis 8 Ringatomen und 1 bis 3 Heteroringatomen aus der Gruppe N, O und S oder ist ein heteroaromatischer Ring mit 5 oder 6 Ringatomen und enthält 1, 2 oder 3 Heteroringatome aus der Gruppe N, O und S. Der Rest kann z.B. ein wie oben definierter heteroaromatischer Rest oder Ring sein oder ist ein partiell hydrierter Rest wie Oxiranyl, Pyrrolidyl, Piperidyl, Piperazinyl, Dioxolanyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie substituierte Kohlenwasserstoffreste, z.B. substitutiertes Alkyl, Alkenyl, Alkinyl, Aryl, Phenyl und Benzyl, oder substituiertes Heteroaryl, ein substituierter bicyclischer Rest, gegebenenfalls mit aromatischen Anteilen, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Cyano, Azido, Alkoxycarbonyl, Alkylcarbonyl, Formyl, Carbamoyl,

Mono- und Dialkylaminocarbonyl, substituiertes Amino wie Acylamino, Mono- oder Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohlenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, $C_1$-$C_4$-Alkyl, vorzugsweise Methyl oder Ethyl, $C_1$-$C_4$-Haloalkyl, vorzugsweise Trifluormethyl, $C_1$-$C_4$-Alkoxy, vorzugsweise Methoxy oder Ethoxy, $C_1$-$C_4$-Haloalkoxy, Nitro und Cyano. Besonders bevorzugt sind dabei die Substituenten Methyl, Methoxy und Chlor.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, $C_1$-$C_4$-Alkyl, $C_1$-$C_4$-Alkoxy, $C_1$-$C_4$-Halogenalkyl, $C_1$-$C_4$-Halogenalkoxy und Nitro substituiert ist, z.B. o-, m- und p-Tolyl, Dimethylphenyle, 2-, 3- und 4-Chlorphenyl, 2-, 3- und 4-Trifluor- und - Trichlorphenyl, 2,4-, 3,5-, 2,5- und 2,3-Dichlorphenyl, o-, m- und p-Methoxyphenyl.

Die Reste Alkyl, Alkoxy, Haloalkyl, Haloalkoxy, Alkylamino und Alkylthio sowie die entsprechenden ungesättigten und/oder substituierten Reste sind im Kohlenstoffgerüst jeweils geradkettig oder verzweigt. Wenn nicht speziell angegeben, sind bei diesen Resten die niederen Kohlenstoffgerüste, z.B. mit 1 bis 4 C-Atomen bzw. bei ungesättigten Gruppen mit 2 bis 4 C-Atomen, bevorzugt. Alkylreste, auch in den zusammengesetzten Bedeutungen wie Alkoxy, Haloalkyl usw., bedeuten, z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyle, 1-Methylhexyl und 1,4-Dimethylpentyl. Cycloalkyl bedeutet einen cycloaliphatischen Kohlenwasserstoffrest wie Cyclopropyl, Cyclobutyl, Cyclopentyl oder Cyclohexyl usw.; Alkenyl, Cycloalkenyl- und Alkinyl haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, Methyl-but-3-en-1-yl und 1-Methyl-but-2-en-1-yl; Cycloalkenyl ist z.B. Cyclopentenyl und Cyclohexenyl; Alkinyl bedeutet z.B. Propargyl, But-2-in-1-yl, But-3-in-1-yl, 1-Methyl-but-3-in-1-yl.

Alkenyl in der Form "($C_3$-$C_4$)Alkenyl" oder "($C_3$-$C_8$)Alkenyl" bedeutet vorzugsweise einen Alkenylrest mit 3 bis 4 bzw. 3 bis 6 C-Atomen, bei dem die Doppelbindung nicht an dem C-Atom liegt, das mit dem übrigen Molekülteil der Verbindung verbunden ist ("yl"-Position). Entsprechendes gilt für ($C_3$-$C_4$)Alkinyl usw.

Halogen bedeutet beispielsweise Fluor, Chlor, Brom oder lod, Haloalkyl, -alkenyl und-alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. $CF_3$, $CHF_2$, $CH_2F$, $CF_3CF_2$, $CH_2FCHCl_2$, $CCl_3$, $CHCl_2$, $CH_2CH_2Cl$; Haloalkyl ist z.B. $OCF_3$, $OCHF_2$, $OCH_2F$, $CF_3CF_2O$, $OCH_2CF_3$ und $OCH_2CH_2Cl$; entsprechendes gilt für Haloalkenyl und andere durch Halogen substituierte Reste.

Mono- oder disubstituiertes Amino bedeutet einen chemisch stabilen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Monoalkylamino, Dialkylamino, Acylamino, Arylamino, N-Alkyl-N-arylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise ($C_1$-$C_4$) Alkanoyl. Entsprechendes gilt für substituiertes Hydroxylamino oder Hydrazino.

Ein Acylrest bedeutet den Rest einer organischen Säure, z.B. den Rest einer Carbonsäure und Reste davon abgeleiteter Säuren wie der Thiocarbonsäure, gegebenenfalls N-substituierter Iminocarbonsäuren, oder der Rest von Kohlensäuremonoestern, gegebenenfalls N-substituierter Carbaminsäure, Sulfonsäuren, Sulfinsäuren, Phosphonsäuren, Phosphinsäuren. Acyl bedeutet beispielsweise Formyl, Alkylcarbonyl wie ($C_1$-$C_4$-Alkyl)-carbonyl, Phenylcarbonyl, wobei der Phenylring substituiert sein kann, z.B. wie oben für Phenyl gezeigt, oder Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren.

Die allgemeinen Formeln umfassen auch Stereoisomere, welche z. B. ein oder mehrere asymmetrische C-Atome oder auch Doppelbindungen enthalten, die in der jeweiligen allgemeinen Formel nicht gesondert angegeben sind. Die durch ihre spezifische Raumform definierten möglichen Stereoisomeren mit gleicher chemischer Verknüpfung, wie Enantiomere, Diastereomere, Z- und E-lsomere sind somit alle von den allgemeinen Formeln umfaßt und können nach üblichen Methoden aus Gemischen der Stereoisomeren erhalten oder auch durch stereoselektiven Reaktionen in Kombination mit dem Einsatz von stereochemisch reinen Ausgangsstoffen hergestellt werden.

Die allgemeinen Formeln umfassen auch Tautomere der bezeichneten Verbindungen, soweit sie durch Protonenwanderung entstehen und soweit sie chemisch stabile Tautomere sind.

Viele der Verbindungen der Formel (I) können Salze bilden, so zum Beispiel diejenigen, bei denen im Fall $R^3$ = H der Wasserstoff der Gruppe -P(=O)($R^2$)(OH) oder auch andere vorhandene acide Wasserstoffatome (z.B. aus Carboxylgruppen u.a.) durch ein für die Landwirtschaft geeignetes Kation ersetzt wird. Diese Salze sind beispielsweise Metallsalze; vorzugsweise Alkali- oder Erdalkalisalze, insbesondere Natrium- und Kaliumsalze, oder auch Ammoniumsalze oder Salze mit organischen Aminen. Ebenso kann Salzbildung durch Anlagerung einer Säure an basische Gruppen, wie z.B. Amino und Alkylamino, erfolgen. Geeignete Säuren hierfür sind starke anorganische und organische

Säuren, beispielsweise HCl, HBr, $H_2SO_4$ oder $HNO_3$.

Der organische Linker in den Verbindungen der Formeln (II) und (IV) bis (IX) hat die Funktion einer Brücke zwischen dem Harzpolymer und dem Molekülteil, das strukturell modifiziert werden soll. Der Linker muß die Bindung des genannten Molekülteils und dessen spätere Abspaltung ermöglichen. Der Linker muß außerdem auf das Harzpolymer aufgebracht werden können, und zwar in der Regel durch eine chemische Reaktion, sofern das Harzpolymer nicht bereits aus geeigneten Monomeren, welche den Linker enthalten, aufgebaut werden kann. Eventuell kann auf einen strukturell besonderen Linker ganz verzichtet werden; in diesem Falle stellt der Linker eine direkte Bindung dar. Als Linker eignen sich strukturell sehr unterschiedliche Reste, die in Abhängigkeit von den Bindungsstellen am Harzpolymer geeignete Bindungsstellen und funktionelle Gruppen aufweisen müssen, wobei im allgemeinen eine Harz-Linker-Verbindung der Formel (X)

$$[\ \text{Harzpolymer}\ ] - [\ \text{Linker} - X\ ]_n \qquad (X)$$

worin [ Harzpolymer ], Linker und n wie in der genannten Formel (II) definiert sind und X eine für den jeweils verwendeten Linker spezifische funktionelle Gruppe bedeutet, zunächst hergestellt oder bereitgestellt wird. Die Harz-Linker-Verbindung (X) wird dann mit einer aromatischen oder heteroaromatischen Verbindung ("Scaffoldsystem") der Formel (XI),

$$Y^1 - E^1 - S^1 \qquad (XI)$$

worin $E^1$ und $S^1$ wie in der genannten Formel (II) definiert sind und $Y^1$ eine funktionelle Gruppe ist, die mit der funktionellen Gruppe X der Harz-Linker-Verbindung zur Brücke Z reagiert, analog bekannten Methoden zum Harz-Linker-Addukt der Formel (II) umgesetzt.

Für das Verfahren sind strukturell sehr unterschiedliche Linker geeignet, beispielsweise auch solche, die aus der harzgebundenen Synthese für die Anbindung von Carbonsäuren, beispielsweise von Aminosäuren bei der Peptidsynthese, eingesetzt werden können.

Verbindungen (Linker-Komponenten), die für den Aufbau des Linkers in Kombination mit einem aminogruppenhaltigen Harz, z.B. einem Aminomethylenpolystyrolharz, oder einem hydroxygruppenhaltigen Harz eingesetzt werden können, sind beispielsweise Linker-Komponenten mit einer Carbonsäuregruppe. Die Herstellung der Harz-Linker-Verbindung der Formel (X) erfolgt dann jeweils durch Reaktion der Carboxygruppe einer Linker-Komponente (XII) mit einer Aminogruppe oder Hydroxygruppe des Harzes (Amidbildung bzw. Esterbildung).

Teilweise können die Linker auch schrittweise hergestellt werden; in einem ersten Schritt wird eine Carbonsäure mit dem aminogruppenhaltigen Harz kondensiert und das erhaltene modifizierte Harz an den eingeführten Gruppen bis zur gewünschten Harz-Linker-Verbindung weiter modifiziert.

Desweiteren sind Harz-Linker-Verbindungen bekannt, welche auf Basis anderer Harze und auf andere Weise aufgebaut werden.

Beispiele für Linker-Komponenten (XII) und Harz-Linker-Verbindungen der Formel (X) sind in den nachfolgenden Tabellen 1 und 2 aufgeführt; eine Linker-Komponente ist jeweils die Verbindung der Formel (XII),

$$W - \text{Linker} - X \qquad (XII)$$

worin W die Abgangsgruppe oder zur Abgangsgruppe zu aktivierende funktionelle Gruppe ist, die bei der Umsetzung mit der funktionellen Gruppe des Harzes, z. B. der Aminogruppe oder Hydroxygruppe des Harzes, ersetzt wird; für den Fall, daß die Harz-Linker-Verbindung (X) anders hergestellt wird oder die Herstellung nicht detailliert angegeben ist, ist der Rest W= Polymer oder $\sim\sim\sim$ angegeben, was die Bindungsstelle der funktionellen Gruppe Linker-X am Harzpolymer andeutet; X hat die weiter oben genannte Bedeutung; Literaturangaben: siehe J.S. Früchtel, G. Jung, Angew. Chem. 108 (1996) 19-46 und dort zitierte Literatur

Tabelle 1: Basenstabile Linker-gruppen zwischen Substrat und fester Phase

| W-Linker-X | Bemerkungen |
| --- | --- |
| 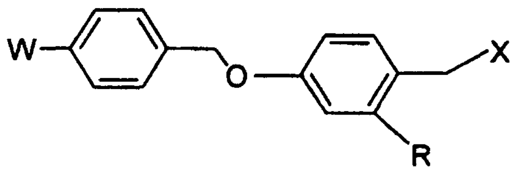<br>W = Polymer, X = OH | a) Wang-Linker (R=H); geeignet zur Fixierung von Carbonsäuren; Abspaltung mit 95%iger Trifluoressigsäure (TFA)<br><br>b) SASRIN-Linker (R = OMe); Fixierung von Carbonsäuren; Abspaltung mit 1%iger TFA |
| 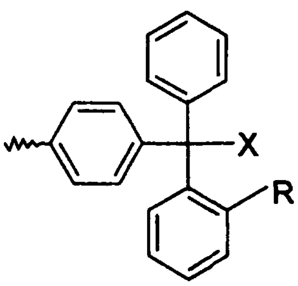 X = Cl | a) Tritylchlorid-Linker (R=H); Fixierung von Nucleophilen, Abspaltung mit schwachen Säuren (HOAc)<br><br>b) 2-Chlortritylchlorid-Linker (R=Cl), Fixierung von Nucleophilen, Abspaltung mit sehr schwachen Säuren wie $HOAc/CH_2Cl_2$ (1/4) |
| 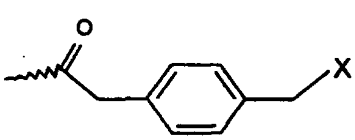 | PAM-Linker; Fixierung von Carbonsäuren, Abspaltung mit HF, TFMSA; |

Tabelle 1 (Fortsetzung):

| W-Linker-X | Bemerkungen |
|---|---|
| 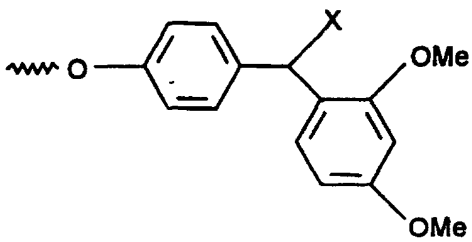 | a) Rink-Säure (X = OH); Fixierung von Carbonsäuren Abspaltung mit $HOAc/CH_2Cl_2$<br>b) Rink-Amid (X = NH-Fmoc); Fixierung von Carbonsäuren als Amid, Abspaltung mit $TFA/CH_2Cl_2$ |
| 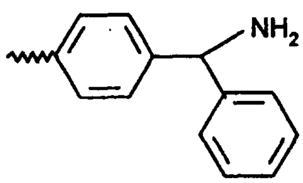 | BHA-Anker; Fixierung von Carbonsäuren; Abspaltung mit TFMSA |
| 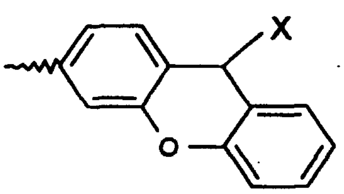<br>X = NH-Fmoc | Sieber-Amid; Fixierung von Carbonsäuren; Abspaltung mit $TFA/CH_2Cl_2$ (1/99)<br>Fmoc = 9-Fluorenyl-methoxy-carbonyl |

Tabelle 2: Säureststabile Linker-gruppen zwischen Substrat und fester Phase

| W-Linker-X | Bemerkungen |
|---|---|
| 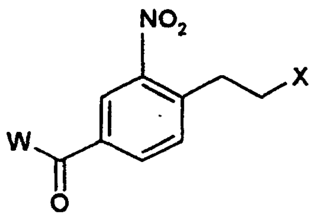 W = OH<br>X = OH | Fixierung von Carbonsäuren<br>Abspaltung mit DBU/Piperidin (ß-Eliminierung); |
| 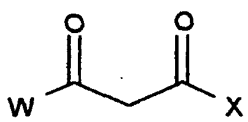 | Fixierung von Alkoholen, Aminen;<br>Abspaltung mit NaOH (Verseifung); |
| 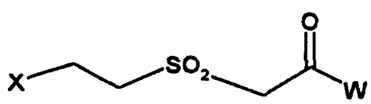<br>W = OH, X = OH | Fixierung von Carbonsäuren; Abspaltung mit NaOH (ß-Eliminierung) |
| 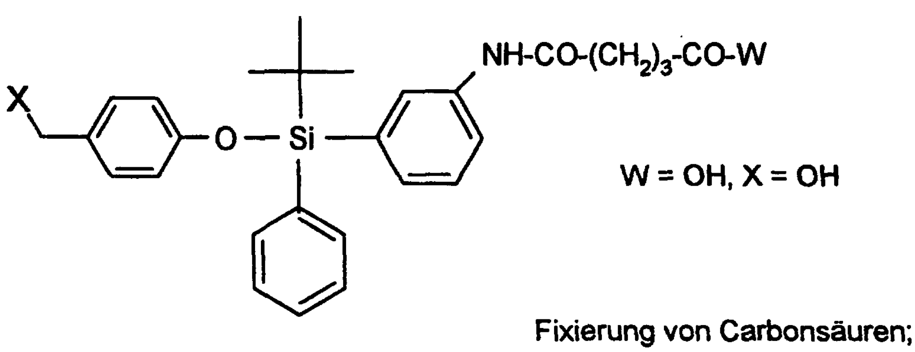 W = OH, X = OH | Fixierung von Carbonsäuren; Abspaltung mit $Bu_4NF$; |

Tabelle 2 (Fortsetzung):

| W-Linker-X | Bemerkungen |
|---|---|
| 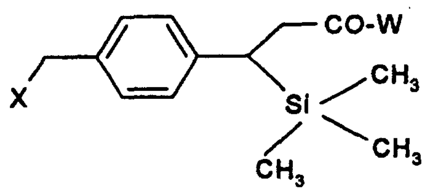 | Fixierung von Carbonsäuren; Abspaltung mit $Bu_4NF$<br>W = OH, X = OH |
| 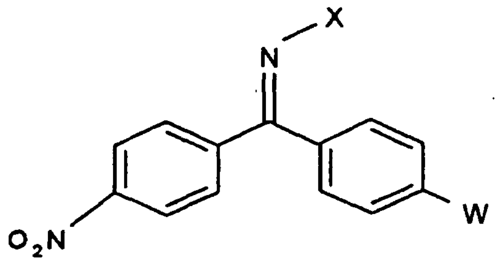 | Fixierung von Carbonsäuren; Abspaltung mit Hydrazinhydrat (Hydrazinolyse); stabil in 25 proz. TFA;<br>W = Polymer, X = OH |
| 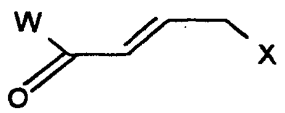<br>W = OH, X = OH | Fixierung von Carbonsäuren; Abspaltung mit $Pd°/H_2$ (katalytische Hydrierung); HYCRAM-Träger |
| 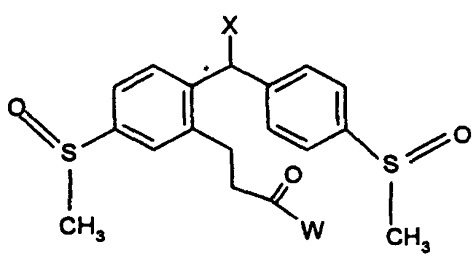 | SCAL-Linker, Fixierung von Carbonsäuren, Abspaltung mit $(EtO)_2P(S)SH$/TFA (reduktive Acidolyse);<br>W = OH, X = $NH_2$ |
| 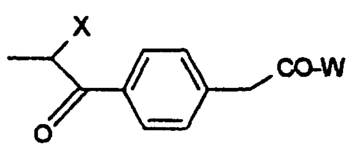<br>W = OH, X = Cl | Fixierung von Carbonsäuren; Abspaltung durch Photolyse (hv = 350 nm. Raumtemp. 72 h); stabil in 50 proz. TFA, labil in Hydrazin-hydrat; |

Tabelle 2 (Fortsetzung):

| W-Linker-X | Bemerkungen |
|---|---|
| 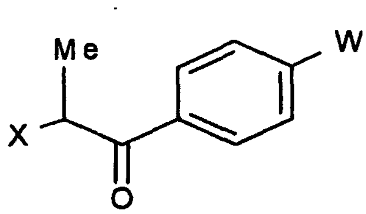 W = Polymer, X = Br | Fixierung von Carbonsäuren; Abspaltung durch Photolyse (hv = 350 nm); labil in Piperidin/DMF; stabiler in Piperidin/$CH_2Cl_2$ |
| 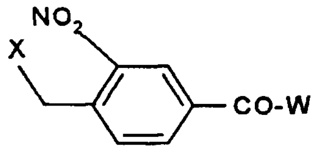 | Fixierung von Carbonsäuren; Abspaltung durch Photolyse (hv = 350 nm, sauerstofffrei unter Inertgas) X = Hal, OH, $NH_2$, W = OH |
| W-CO-p-$C_6H_4$-S-$CH_2CH_2$-X | Rydon-Linker; P. M. Hardy, H. N. Rydon, R. C. Thompson, Tetrahedron Lett. 1968, 2525-2526 X = OH, W = OH |

Die verwendbaren Harzpolymere sollen in den flüssigen Phasen, die für die Reaktionen und Isolierung der Verbindungen eingesetzt werden, unlöslich, weitgehend inert gegenüber den Reaktionsbedingungen in Stufen a) bis g) und filtrierbar sein; jedes Harzpolymerpartikel weist vorzugsweise viele Bindungsstellen für die jeweiligen Linker auf. In Abhängigkeit von der Struktur der gewählten Linker kommen vom Aufbau her gänzlich unterschiedliche Harzpolymere in Frage, beispielsweise Polystyrolharze, Polyamidharze, Polydimethylacrylamidharze, modifizierte Harze auf Basis der genannten Harze und Mischpolymere. Bevorzugte Harze sind Aminomethylenpolystyrolharze, d. h. aminomethylierte Polystyrolharze, oder auch anders modifizierte Harze auf Polystyrolbasis, z. B. Pfropfpolymerisate von Polystyrol und Polyethylenglykol wie solche aus der Reihe ®TentaGel (Fa. Rapp Polymere, Tübingen, Deutschland), in Form quellfähiger Partikel im Korngrößenbereich von beispielsweise 0,01 bis 1 mm, vorzugsweise 0,05 bis 0,5 mm, und einer Beladung von Aminomethylgruppen von 0,01 bis 10 mmol pro Gramm Harz, vorzugsweise 0,1 bis 2 mmol pro Gramm Harz.

Die einzelnen Linker werden in an sich bekannter Weise auf das Harz aufgebracht; siehe genannte Literatur zu Tabellen 1 und 2. Dabei können unterschiedlichste Techniken eingesetzt werden. Geeignete Linker-Komponenten für die Kombination mit den hydroxy- oder aminomethylierten Polystyrolharzen sind Linker mit Carbonsäuregruppen, die unter üblichen Bedingungen für Kondensationen und speziell für Ester- und Amidbildungsreaktionen umgesetzt werden. Geeignet sind schonende Methoden bei moderaten Temperaturen. Die Umsetzung kann z. B. in einem weitgehend wasserfreien inerten organischen Lösungsmitteln in Gegenwart von Katalysatoren oder üblichen Kondensationsmitteln bei Temperaturen von beispielsweise -30°C bis 200°C, vorzugsweise von 0°C bis 150°C, insbesondere 0°C bis 100°C durchgeführt werden. Abhängig vom jeweiligen Harz können auch wäßrig-organische Lösungsmittel eingesetzt werden.

Mit der Bezeichnung "inertes Lösungsmittel" sind Lösungsmittel gemeint, die unter den jeweiligen Reaktionsbedingungen inert sind, jedoch nicht unter beliebigen Reaktionsbedingungen inert sein müssen. Für die obengenannte Kondensation kommen beispielsweise folgende in Frage:

- Ether wie tert.-Butylmethylether, Dimethoxyethan (DME), Tetrahydrofuran (THF), Diethylether, Diisopropylether,
- dipolar aprotische Lösungsmittel wie Dimethylformamid (DMF), N,N-Dimethylacetamid (DMA), N-Methyl-pyrrolidon (NMP), Acetonitril,
- gegebenenfalls halogenierte aliphatische oder aromatische Kohlenwasserstoffe wie Dichlormethan, Toluol, o-Chlortoluol, Chlorbenzol, oder
- Gemische aus inerten Solventien.

Als Kondensationsmittel zur Herstellung der Harz-Linker-Verbindung (X) aus der Linker-Komponente (XII) und einem Hydroxymethylen- oder Aminomethylenpolystyrolharz eignen sich übliche Mittel wie azeotrope Destillation, Umsetzung mit aktivierten Derivaten der Carbonsäure wie Halogeniden oder aktiven Estern. Besonders geeignet sind schonende Methoden wie die Umsetzung in Gegenwart von Carbodiimiden wie Dicyclohexylcarbodiimid (DCC) oder Diisopropylcarbodiimid.
Nach Herstellung der oben beschriebenen [Harzpolymer]-Linker-X-Strukturen (X) werden diese mit Verbindungen der Formel (XI) zu Verbindungen der Formel (II) umgesetzt. Hierbei handelt es sich beispielsweise um Verbindungen (X) mit X = OH oder $NH_2$, die mit Carbonsäuren der Formel (XI) ($Y^1$ = -COOH) zu harzgebundenen Estern oder Amiden (Z = -O-CO- bzw. -NH-CO-) umgesetzt werden.
Die so zugänglichen gegebenenfalls substituierten (hetero)aromatischen Addukte der Formel (II) müssen eine funktionelle Gruppe ($S^1$) tragen, die es ermöglicht, Pd-katalysierte Reaktionen zum Aufbau von (Hetero)aryl-Phosphor-III-Verbindungen durchzuführen (vgl. analoge Bedingungen der Heck-Reaktion; siehe Lit. R.F. Heck, Palladium, Reagents in Organic Synthesis, Academic Press 1985).

Ein zentraler Schritt des erfindungsgemäßen Verfahrens ist der Aufbau einer Arylphosphorverbindung (Phosphor-III-Verbindung) auf der festen Phase (Stufe a), bei der unter Bedingungen analog der Heck-Reaktion Derivate der unterphosphorigen Säure der Formel (III) eingesetzt werden.

Aus der Literatur sind bereits Verfahren dieses Typs bekannt, bei denen man Phosphinate wie $H_2PO_2CH_3$ oder $H_2PO_2C_2H_5$ einsetzt; siehe Palladium Reagents and Catalysts, Innovations in Organic Synthesis, Jiro Tsuji, John Wiley & Sons, Chichester England 1995, S. 243 ff, desweiteren in Lit. Haiyan Lei, Mark S. Stoakes, Kamal P.B. Herath, Jinho Lee and Alan W. Schwabacher, J. Org. Chem. 59 (1994), 4206-4210, desweiteren in Lit. Haiyan Lei, Mark. S. Stoakes, Alan W. Schwabacher, Synthesis 1992, S. 1255-1260.

Typische Beispiele für eine Gruppe, mit der palladiumkatalysierte Reaktionen der oben angegebenen Art durchgeführt werden können, sind organische Halogenverbindungen, vorzugsweise Jodide und Bromide, aber auch Pseudohalogene wie Triflate oder Tosylate und andere; siehe z.B. analoge Reaktionen und Reaktionsbedingungen in R.F. Heck, Palladium Reagents in Organic Synthesis, Academic Press, 1985. Im erfindungsgemäßen Verfahren kann vorzugsweise von lodiden aber auch von Bromiden ausgegangen werden. Pseudohalogenide, z.B. Triflate oder Tosylate, lassen sich aus geeigneten Vorstufen, z. B. Phenolen, herstellen. Dieser Aufbau von Pseudohalogeniden kann auf fester Phase durchgeführt werden.

Analog der literaturbekannten Heck-Reaktion kann das Palladium in Form von Pd-II-Salzen eingesetzt werden, beispielsweise Bis-triphenylphosphan-$Pd^{II}$-dichlorid, die in situ einen reaktiven $Pd^0$-Komplex bilden. Alternativ können auch $Pd^0$-Komplexe wie Tetrakis-triphenylphosphan-Pd u. a. eingesetzt werden.

Nach Durchführung der oben genannten Verfahren zur palladiumkatalysierten Einführung einer Phosphor-Kohlenstoffbindung erhält man Verbindungen der allgemeinen Formel (IV) oder, nach Derivatisierung der Gruppe $E^1$ zu $(E^1)'$, Verbindungen der Formel (IV)' als gegebenenfalls substituierte (hetero)aromatische Phosphonigsäureester, vorzugsweise mit niederen Alkylgruppen $A^1$ im Ester, beispielsweise $C_1$-$C_8$-Alkyl, insbesondere $C_1$-$C_5$-Alkyl.

Ein weiterer, optionaler Schritt der Erfindung besteht in der Hydrolyse der Phosphonigsäureester an fester Phase zu phosphonigen Säuren (V) bzw (V)'. Hierzu wird in einem Lösungsmittel, in dem das Harzpolymer quellfähig ist und das unter den Reaktionsbedingungen inert ist, beispielsweise die weiter oben allgemein erwähnten Lösungsmittel, vorzugsweise Tetrahydrofuran (THF), Dioxan oder Ethylenglycoldiether, eine starke organische Base, wie z.B. 1,8-Diazabicyclo[5.4.0]undec-7-en unter Zusatz einer geeigneten Menge Wasser, beispielsweise 1 bis 100 Äquivalente, vorzugsweise 1 bis 10 Äquivalente, bei Temperaturen von 0 bis 100°C, vorzugsweise 10°C bis 50°C umgesetzt. Die entstehenden Phosphonigsäuren können dann an fester Phase zu Aktivestem umgesetzt werden, z.B. durch Reaktion mit Pivaloylchlorid in Acetonitril/Pyridin (Lit. zu analogen Reaktionen, siehe B. C. Froehler; M. D. Matteucci, Tetrahedron Lett. 27 (1986), 469), und diese mit praktisch beliebigen Alkoholen $R^3$-OH zu einer Vielzahl von Phosphonigsäurestem zu Verbindungen der Formel (VI) bzw. (VI)' umgesetzt werden.
Weitere geeignete Veresterungsmethoden für Phosphonigsäuren sind in folgenden Literaturstellen beschrieben:

- Xiadong Cao, A.M.M. Mjalli, Tetrahedron Letters 37 (1996) 6073-6076,
- Changzhi Zhang, A. M.M. Mjalli, Tetrahedron Letters 37 (1996) 5457-5460,

Grundsätzlich wird durch den Schritt der Hydrolyse und der Veresterung eine breite Vielfalt des Restes $R^3$ in Formel (I) zugänglich.

Gegenstand der Erfindung sind insbesondere auch die harzgebundenen Verfahren zur Umsetzung von Addukten der Formeln (IV) bis (VI) bzw. (IV)' bis (VI)', deren gemeinsames Strukturmerkmal Phosphonigsäure- oder Phosphonigsäuremonoester-gruppen ist, mit einer Reihe von Verbindungen mit funktionellen Gruppen, die unter Ausbildung von Phosphor-Kohlenstoff-Bindungen an die oben genannten Phosphor-III-Verbindungen addieren (Einführung des Restes $R^2$).

Beispielsweise können die genannten Verbindungen, vorzugsweise nach Umsetzung der Phosphorkomponente mit Silylierungsreagentien wie z.B. Trimethylsilylchlorid, Triethylamin, Bistrimethylsilylacetamid, oder Hexamethyldisilazan oder auch Gemischen der Silyierungsreagentien, aktiviert und mit Elektrophilen, beispielsweise Aldehyden, Iminen, Isocyanaten oder Michael-Akzeptoren, in die entsprechenden Produkte der Formel (VII) und (VIII) bzw. (VII)' bis (VIII)' umgesetzt werden; siehe analoge Methoden zur Silylierung in:

- Kamyar Afarinkia, Charles W. Rees, Tetrahedron 46 (1990) 7175-7196;
- E.A. Boyd A.C. Reagan, Tetrahedron Letters 35 (1994), 4223-4226;
- J.K Thottathil, O.E. Ryono, C.A. Przybyla, J.L. Moniot, R. Neubeck Tetrahedron Lett. 25 (1984) 4741-4744;
- O.A. Evans, K Hurst, J.M. Jakaes, J.Am Chem. Soc. 100 (1978) 3467;
- K Issleib et al., DD-Patent 24 28 10,

Die Verbindungen der allgemeinen Formel (IV) bis (VI) bzw. (IV)' bis (VI)' können auch basenkatalysiert mit den oben genannten Elektrophilen zur Reaktion gebracht werden. Als Basen kommen neben anorganischen Salzen, wie z.B. Kalium-tert.-butylat, vor allem organische Stickstoffbasen, wie z.B. Triethylamin, 1,8-Diazabicyclo[5.4.0]undec-7-en, N-Isopropyl-N-ethylamin und ähnliche Verbindungen in Betracht (Lit: R.B. Fox, W. J. Bailey, J.Org. Chem. 25 (1960) 1447; siehe auch Lit. Houben-Weyl, "Methoden der Org. Chemie", Georg Thieme Verlag 1963, Bd. 12/1, "Organische Phosphorverbindungen").

Die Auswahl der geeigneten Reaktionssequenz zur Aktivierung der Verbindungen der allgemeinen Formel (IV) bis (VI) bzw. (IV)' bis (VI)' hängt von den spezifischen chemischen Reaktivitäten weiterer funktioneller Gruppen in $E^1$, $(E^1)'$ oder $R^1$ ab und gibt dem Fachmann auch die Möglichkeit vielseitige chemische Umsetzungen an $E^1$ bzw. $(E^1)$ ' durchzuführen. Die entstandenen Phosphinsäurederivate der allgemeinen Formel (VII) bis (VIII) bzw. (VII)' bis (VIII) ' sind ebenfalls ausreichend stabil um vielseitige chemische Umsetzungen an den Resten $E^1$, $(E^1)'$ und $E^2$ durchzuführen. Die Reste $E^1$, $(E^1)'$ und $E^2$ müssen dabei so ausgewählt werden, daß eine Modifizierung der Reste zu den Resten $R^1$ bzw. $R^2$ in Formel (I) möglich ist.

Auf die beschriebene Weise lassen sich vorzugsweise Verbindungen der Formel (I) herstellen, worin

$R^1$ Phenylen bedeutet, das unsubstituiert oder durch einen bis vier Reste aus der Gruppe Halogen, Alkyl, Haloalkyl, Alkoxy, Haloalkoxy, Alkylthio, Hydroxy, Amino, Nitro, Cyano, Azido, Alkoxycarbonyl, Alkylcarbonyl, Formyl, Carbamoyl, Mono- und Dialkylaminocarbonyl, Acylamino, vorzugsweise Alkanoylamino mit 1 bis 6 C-Atomen, Mono- und Dialkylamino, Alkylsulfinyl, Haloalkylsulfinyl, Alkylsulfonyl und Haloalkylsulfonyl substituiert ist, wobei jeder Substituent bis zu 6 C-Atome im Alkylteil haben kann, oder einen heteroaromatischen Rest aus der Gruppe der Fünf- oder Sechsringe mit jeweils 1, 2 oder 3 Heteroatomen aus der Gruppe N, O und S bedeutet, wobei der Rest unsubstituiert oder durch einen bis vier Reste aus der Gruppe Halogen, Alkyl, Haloalkyl, Alkoxy, Haloalkoxy, Alkylthio, Hydroxy, Amino, Nitro, Cyano, Azido, Alkoxycarbonyl, Alkylcarbonyl, Formyl, Carbamoyl, Mono- und Dialkylaminocarbonyl, substituiertes Amino wie Acylamino, vorzugsweise Alkanoylamino mit 1 bis 6 C-Atomen, Mono- und Dialkylamino, Alkylsulfinyl, Haloalkylsulfinyl, Alkylsulfonyl und Haloalkylsulfonyl substituiert ist und jeder Substituent vorzugsweise bis zu 6 C-Atome, insbesondere 4 C-Atome im Alkylteil haben kann, und

$R^2$ Wasserstoff, einen aliphatischen Kohlenwasserstoffrest, der unsubstituiert oder substituiert ist und inklusive Substituenten 1 bis 30 C-Atome, vorzugsweise 1 bis 20 C-Atome enthält, bedeutet,

$R^3$ Wasserstoff oder einen aliphatischen Kohlenwasserstoffrest, der unsubstituiert oder substituiert ist und inklusive Substituenten 1 bis 30 C-Atome, vorzugsweise 1 bis 20 C-Atome enthält, bedeutet, oder einen Aryl oder Heteroarylrest, der unsubstituiert oder substituiert ist und inklusive Substituenten 1 bis 30 C-Atome enthält, bedeutet, vorzugsweise Alkyl, Alkenyl, Alkinyl mit jeweils 1 bis 12 C-Atomen, wobei jeder der 3 vorstehenden Reste unsubstituiert oder durch einen oder mehrere Reste aus der Gruppe Halogen, Haloalkyl, Alkoxy, Haloalkoxy, Alkylthio, Nitro, Cyano, Alkoxycarbonyl, Alkylcarbonyl und unsubstituiertes und substituiertes Phenyl substituiert ist, wobei jeder Substituent bis zu 4 C-Atome im Alkylteil haben kann, und

Y H, COOH, $CONH_2$, OH, $NH_2$ oder Alkylamino bedeutet.

Von besonderem Interesse sind Verbindungen (I) mit $R^3$ = ($C_1$-$C_4$)Alkyl und Y = COOH.

Bevorzugt sind Verbindungen der Formel (I) bzw. deren erfindungsgemäße Herstellung, worin zwei oder mehrere der Reste $R^1$ bis $R^3$ und Y jeweils eine der bereits genannten oder weiter unten genannten bevorzugten Bedeutungen haben.

Bei der Derivatisierung an der Phosphonigsäure(ester)gruppe lassen sich unterschiedlichste Verfahren mit elektrophilen Reaktionspartnern anwenden; beispielsweise ist es meist möglich, folgende Substanzklassen mit Verbindungen der Formel (IV) reagieren zu lassen:

1) Aldehyde reagieren zu a-Hydroxyphosphinsäurederivaten

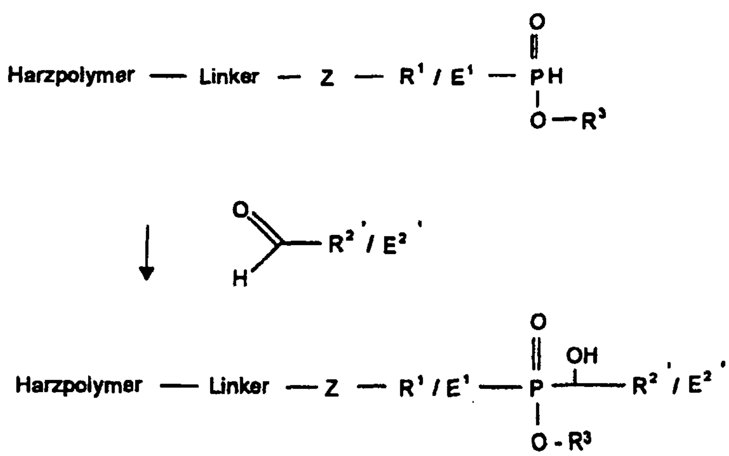

2) Imine reagieren zu subst. $\alpha$-Aminophosphinsäurederivaten

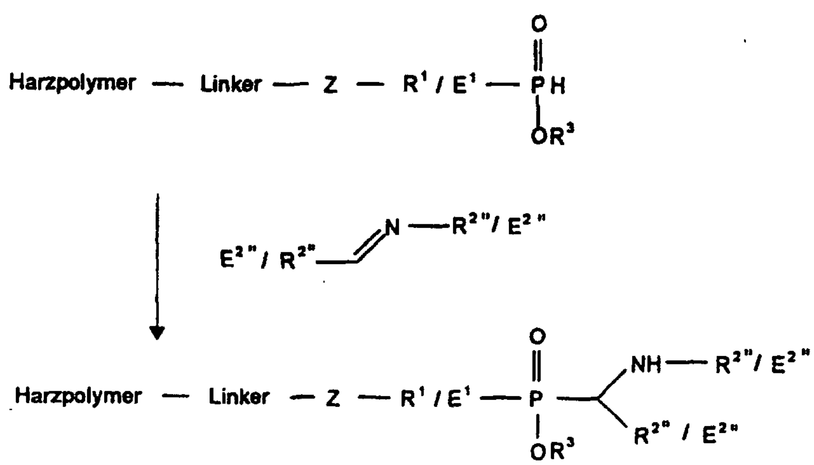

3) Isocyanate reagieren zu substituierten Aminoacylphosphinsäurederivaten

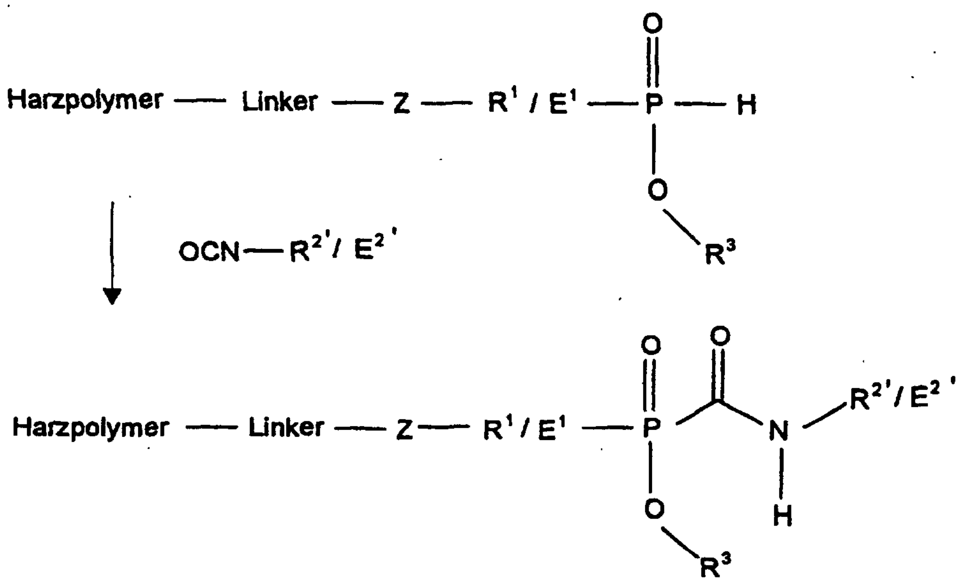

4) Michael-Akzeptoren reagieren zu substituierten Ethylphosphinsäurederivaten (V = elektronegative Gruppe)

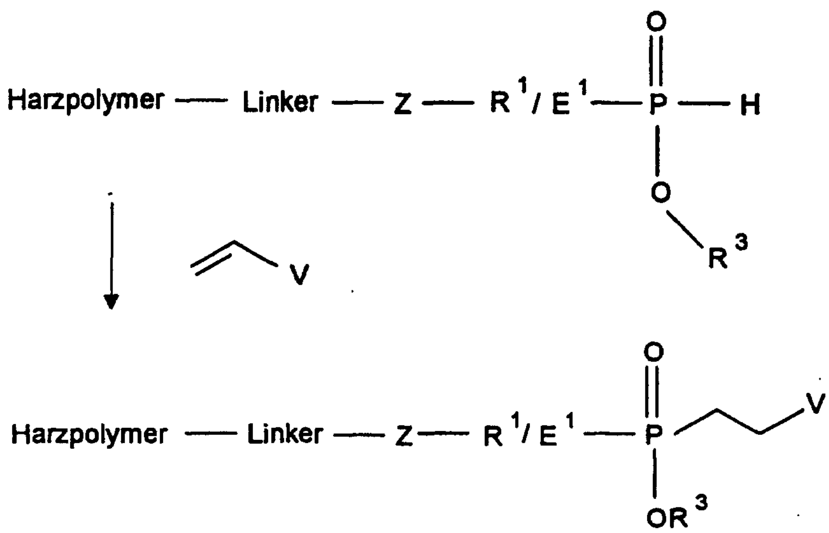

5) Halogenverbindungen reagieren zu Phosphinsäuren bzw. deren Estern (Typ Arbuzov-Reaktion)

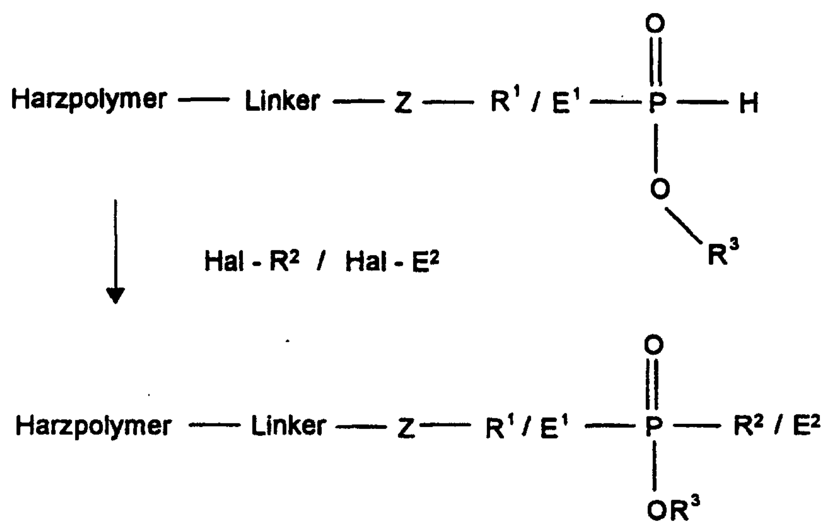


Die in den Formeln der Reaktionsschemata 1) bis 5) gezeigten Reste $R^{2'}$ und $E^{2'}$, $R^{2''}$ und $E^{2''}$ sind nur Teile derjenigen Reste $R^2$ und $E^2$ in Verbindungen der Formeln (VII) und (VIII) bzw. (VII)' und (VIII)', die bei der jeweiligen Reaktion am Phosphoratom eingeführt werden. Die übrigen Teile der Reste $R^2$ und $E^2$ bestehen in den funktionellen Gruppen der Reaktionspartner und sind zur Verdeutlichung in den Reaktionsschemata explizit aufgezeigt worden. Es ist deshalb zu beachten, daß die Reste $R^{2'}$ und $E^{2'}$, $R^{2''}$ und $E^{2''}$ nicht mit den Resten $R^2$ und $E^2$ in Formeln (VII) und (VIII) bzw. (VII)' und (VIII)' identisch sind.

Für alle Reaktionstypen 1) bis 5) können die Phosphor-III-Verbindungen der allgemeinen Formeln (IV) beispielsweise durch Zusatz einer geeigneten Base, wie z.B. Triethylamin, 1,8-Diazabicyclo[5.4.0]undec-7-en und Diisopropylethylamin, deprotoniert oder durch Silylierungen aktiviert und anschließend mit den obengenannten geeignet funktionalisierten Verbindungen umgesetzt werden. Entsprechende Reaktionen sind auch mit den Verbindungen der Formeln (V) und (VI) bzw. (IV)' bis (VI)' durchführbar.

Geeignete Reagentien zur Silylierung der obengenannten Verbindungen sind beispielsweise Trialkylsilylchloride/Trialkylamine, Hexamethyldisilazan, Bistrimethylsilylacetat und weitere dem Fachmann bekannte Silylierungsreagentien.

Bei der Wahl der Aktivierungsreaktionen durch Deprotonierung oder Silylierung sind mögliche Wechselwirkung zwischen funktionellen Gruppen in den organischen Resten $E^1$, $R^1$, $(E^1)'$, $E^2$ und $R^2$ in der dem Fachmann bekannten Weise zu berücksichtigen. Eine generelle Einschränkung der Reste in den Verbindungen vom Typ (IV), (V) oder (VI), (VII) bzw. (IV)', (V)' oder (VI)' besteht nicht.

Derivatisierungsreaktionen oder Reaktionen, bei denen die oben beschriebenen Reste $E^1$ oder $E^2$ zu Resten $R^1$ oder $R^2$ umgesetzt werden, sind in der Regel vom Typ her bekannt und können meist unter analogen Reaktionsbedingungen angewendet werden, wobei manche bevorzugten Verfahrensmaßnahmen durch Besonderheiten des Harzkörpers bedingt sind. Bei der Wahl der Derivatisierungsreaktionen sind mögliche Wechselwirkungen zwischen funktionellen Gruppen in den organischen Resten $E^1$ und $E^2$ bzw. $(E^1)'$ und $(E^2)'$ bzw. $R^1$ und $R^2$ in der dem Fachmann bekannten Weise zu berücksichtigen. Eine generelle Einschränkung der organischen Reste in den Verbindungen (VII), (VIII) oder (IX) bzw. (VII)' oder (VIII)' besteht nicht.

Auf die beschriebene Weise lassen sich übersichtlich strukturbezogene Substanzbibliotheken (Libraries) herstellen, die für die systematische Prüfung der enthaltenen einzelnen Verbindungen oder deren Mischungen auf biologische oder physikalisch-technische Eigenschaften besonders geeignet sind. Eine Substanzbibliothek kann dabei in Abhängigkeit von der Zahl der Ausgangsverbindungen unterschiedlicher Struktur und der Zahl unterschiedlicher Reaktionen und Reaktionsschritte eine bis beliebige viele Verbindungen enthalten. Insbesondere ist durch die Struktur der Ausgangsverbindungen, der Reaktionen und der Reaktionsabfolge jede der Verbindungen in einer Substanzbibliothek strukturell definiert. Gegenstand der Erfindung sind deshalb auch die neuen Verbindungen, die in den erfindungsgemäßen Substanzbibliothekn enthalten sind.

Ein Beispiel für die Herstellung einer systematischen Substanzbibliothek für Testverbindungen ist in Schema 1 (siehe nächste Seite) dargestellt.

Schema 1

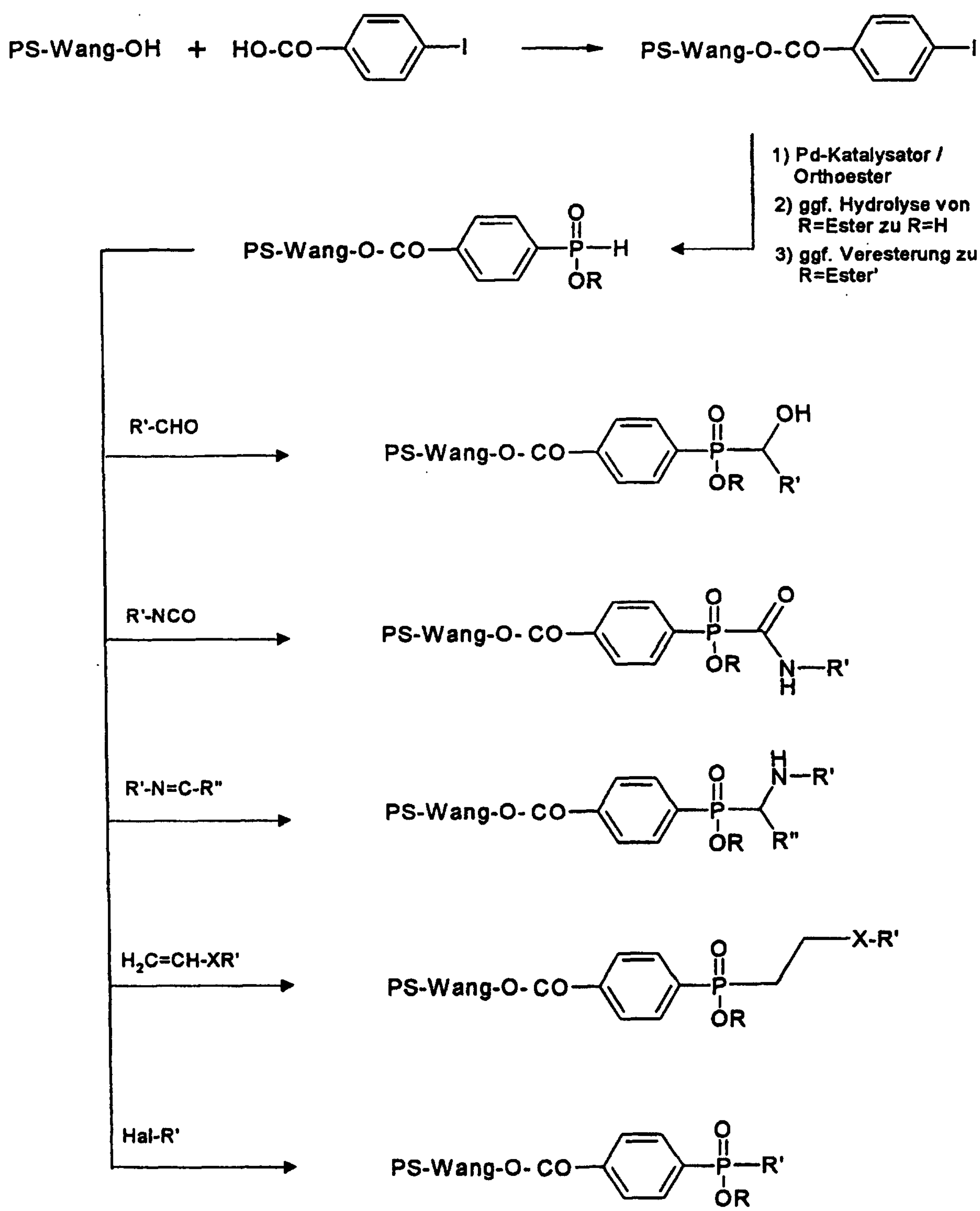


Gemäß Schema 1 wird nach der Anbindung einer Jodbenzoesäure (hier am Beispiel der p-Iod-benzoesäure als Verbindung (XI)) an die Harz-Linker-Verbindung (X) (hier am Beispiel einer Polystyrol-Wang-Linker-Verbindung) das p-Iodbenzolcarbonyloxy-Harz-Linker-Addukt (= Verbindung (II)) erhalten, welches mit einem Orthoameisensäurealkylester unter Pd-Katalyse das entsprechende Benzolphosphonigsäure-Harz-Linker-Addukt (= Verbindung (IV)) ergibt. Das erfindungsgemäße harzgebundene Zwischenprodukt der Formel (II) wird anschließend benutzt, um durch Additions- und Substitutionsreaktionen mit einer Serie von Aldehyden, Iminen, Isocyanaten, Michaelakzeptoren und Organohalogenverbindungen entsprechende Untergruppen von Phosphinsäureesterderivaten der gezeigten Art herzustellen (Schema 1, R = Alkyl).

Wird vor der Umsetzung der Verbindung (IV) die Phosphonigsäureestergruppe zur Verbindung der Formel (V) hydrolysiert, ergibt sich eine entsprechende Substanzbibliothek mit harzgebundenen freien Phosphinsäuren (Schema 1, R = H). Ebenso erhält man nach erneuter Veresterung der Verbindung (V), z. B. mit Benzylalkohol zur Verbindung des Typs (VI), eine entsprechende Substanzbibliothek mit harzgebundenen freien Phosphinsäurebenzylestern (Schema 1, R = $CH_2C_6H_5$).

Mit analogen Umsetzungen unter Verwendung anderer Harz-Linker-Systeme gemäß Tabelle 1, z. B. mit einem Polystyrol-Rink-amid-Linker, lassen sich die harzgebundenen Produkte ebenfalls herstellen.
Im Anschluß an die Umsetzungen gemäß Schema 1 und analogen Derivatisierungen werden die harzgebundenen Produkte ("Scaffold") zweckmäßig durch Trennung der Linker-Scaffoldbindung abgespalten. Die Abspaltbedingungen hängen vom einzelnen Linker ab und sind in der Regel literaturbekannt oder können in Vorversuchen optimiert werden.
Die Abspaltung führt zu den gewünschten Syntheseprodukten der allgemeinen Formel (I). Im Falle des Wang-Linkers und der Arbuzov-Reaktion nach Schema 1, unterste Reihe, erhält man nach Abspaltung mit Trifluoressigsäure als Produkt einen (p-Carboxyphenyl)(R')phosphinsäure-alkylester der Formel (Ia),

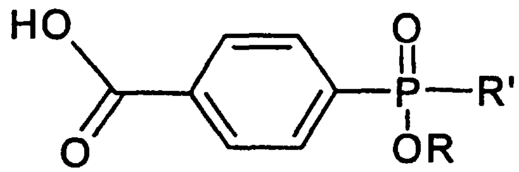

(Ia)

worin R' wie $R^2$ definiert ist, ausgenommen Wasserstoff.

Die beschriebenen Verfahrensvarianten ermöglichen ein breite Wahl für die Struktur des Restes $R^2$ in Verbindungen der Formel (I). Von besonderem Interesse sind daher Verbindungen (I) und die genannten harzgebundenen Vorstufen, worin $R^2$ Wasserstoff oder einen aliphatischen acyclischen oder cyclischen Kohlenwasserstoffrest mit 1 bis 20 C-Atomen oder Heterocyclyl mit 3 bis 7 Ringatomen und 1, 2 oder 3 Heteroatomen aus der Gruppe N, O und S bedeutet, wobei der Kohlenwasserstoffrest oder der Heterocyclylrest jeweils unsubstituiert oder durch einen oder mehrere Reste aus der Gruppe Halogen, Alkoxy, Alkenyloxy, Alkinyloxy, Haloalkoxy, Haloalkenyloxy, Haloalkinyloxy, Alkylthio, Amino, Nitro, Cyano, Azido, Alkoxycarbonyl, Alkenyloxycarbonyl, Alkinyloxycarbonyl, Alkylcarbonyl, Alkenylcarbonyl, Alkinylcarbonyl, Formyl, Carbamoyl, Mono- und Dialkylaminocarbonyl, Acylamino, Mono- und Dialkylamino, Alkylsulfinyl, Haloalkylsulfinyl, Alkylsulfonyl und Haloalkylsulfonyl, unsubstituiertes und substituiertes Cycloalkyl, unsubstituiertes und substituiertes Cycloalkenyl, unsubstituiertes und substituiertes Aryl, unsubstituiertes und substituiertes Heterocyclyl, unsubstituiertes und substituiertes Cycloalkoxy, unsubstituiertes und substituiertes Cycloalkenyloxy, unsubstituiertes und substituiertes Aryloxy, unsubstituiertes und substituiertes Heterocyclyloxy, unsubstituiertes und substituiertes Cycloalkylamino, unsubstituiertes und substituiertes Cycloalkenylamino, unsubstituiertes und substituiertes Arylamino, unsubstituiertes und substituiertes Heterocyclylamino, und im Falle cyclischer Reste auch Alkyl und Haloalkyl substituiert ist.

Dabei sind Reste mit kürzerkettigen Kohlenwasserstoff-(Alkyl-)teilen bevorzugt, z. B. mit 1 bis 8 C-Atomen, vorzugsweise 1 bis 6 C-Atomen, insbesondere mit 1 bis 4 C-Atomen.

Bevorzugt sind auch Verbindungen (I) und deren harzgebunden Vorstufen, worin

$R^2$ einen Rest der Formel ($R^2$a), ($R^2$b), ($R^2$c), ($R^2$d) oder ($R^2$e) bedeutet,

-CHOH-R* ($R^2$a)

-CO-NH-R* ($R^2$b)

-CHR**-NH-R* ($R^2$c)

$$-CR^aR^b-CR^cR^d-X-R^e \qquad (R^2d)$$

$$-R^* \qquad (R^2e)$$

worin

| | |
|---|---|
| R* | einen einen aliphatischen acyclischen oder cyclischen Kohlenwasserstoffrest mit 1 bis 12 C-Atomen oder Heterocyclyl mit 3 bis 6 Ringatomen und 1, 2 oder 3 Heteroatomen aus der Gruppe N, O und S bedeutet, wobei der Kohlenwasserstoffrest oder der Heterocyclylrest jeweils unsubstituiert oder durch einen oder mehrere Reste aus der Gruppe Halogen, Alkoxy, Alkenyloxy, Alkinyloxy, Haloalkoxy, Haloalkenyloxy, Haloalkinyloxy, Alkylthio, Amino, Nitro, Cyano, Azido, Alkoxycarbonyl, Alkenyloxycarbonyl, Alkinyloxycarbonyl, Alkylcarbonyl, Alkenylcarbonyl, Alkinylcarbonyl, Formyl, Carbamoyl, Mono- und Dialkylaminocarbonyl, Acylamino, vorzugsweise Alkanoylamino, Mono- und Dialkylamino, Alkylsulfinyl, Haloalkylsulfinyl, Alkylsulfonyl, Haloalkylsulfonyl, unsubstituiertes und substituiertes Cycloalkyl, unsubstituiertes und substituiertes Cycloalkenyl, unsubstituiertes und substituiertes Aryl, unsubstituiertes und substituiertes Heterocyclyl, unsubstituiertes und substituiertes Cycloalkoxy, unsubstituiertes und substituiertes Cycloalkenyloxy, unsubstituiertes oder substituiertes Aryloxy, unsubstituiertes und substituiertes Heterocyclyloxy, unsubstituiertes und substituiertes Cycloalkylamino, unsubstituiertes und substituiertes Cycloalkenylamino, unsubstituiertes und substituiertes Arylamino, unsubstituiertes oder substituiertes Heterocyclylamino und im Falle cyclischer Reste auch Alkyl und Haloalkyl substituiert ist, |
| R** | ein Rest aus der Gruppe der für R* definierten Reste ist oder |
| R* und R** | gemeinsam eine Alkylenbrücke bedeuten, welche unsubstituiert oder durch einen oder mehrere Reste substituiert ist, die unabhängig voneinander aus der Gruppe der Substituenten am Kohlenwasserstoffrest für R* ausgewählt sind, und |
| $R^a$, $R^b$, $R^c$, $R^d$, $R^e$ | unabhängig voneinander jeweils einen Rest aus der Gruppe der für R* definierten Reste ist oder |
| $R^a$, $R^c$ oder $R^d$, oder $R^c$, $R^e$ | paaweise eine Alkylenbrücke bedeuten, welche unsubstituiert oder durch einen oder mehrere Reste substituiert ist, die unabhängig voneinander aus der Gruppe der Substituenten am Kohlenwasserstoffrest für R* ausgewählt sind. |

Dabei sind Reste mit kürzerkettigen Kohlenwasserstoff-(Alkyl-)teilen bevorzugt, z. B. mit 1 bis 8 C-Atomen, vorzugsweise 1 bis 6 C-Atomen, insbesondere mit 1 bis 4 C-Atomen.

Bei den allgemeinen Begriffen wie "substituiertes Cycloalkyl, Aryl, Heterocyclyl" sind als Substituenten vorzugsweise die weiter oben bei der allgemeinen Definition substituierter Reste genannten bevorzugten Substituenten von besonderem Interesse.

Weitere Variationsmöglichkeiten zur Herstellung von Verbindungen der Formel (I) ergeben sich aus der Möglichkeit, bereits Modifikationen an den harzgebundenen Vorstufen der Formel (II) vorzunehmen. Eine Derivatisierung der Verbindung (II) und deren Weiterverarbeitung sind in Schema 2 (siehe nächste Seite) an einem Beispiel angedeutet.

Gemäß Schema 2 wird beispielsweise 5-Jodanthranilsäure an ein Harzpolymer, das den Wang-Linker trägt, angeknüpft. Sie enthält eine funktionelle Gruppe (Aminogruppe), die zu Derivaten umgesetzt werden kann. Anstelle der Einführung über die Anthranilsäure kann die Aminogruppe auch durch Reduktion einer Nitrogruppe hergestellt werden. Für die Reduktion eignen sich viele der für Nitrogruppen geeigneten chemischen Reduktionsmittel, wie Metallsalze unter sauren Bedingungen, vorzugsweise milde Reduktionsmittel, die in organischen Lösemitteln eingesetzt werden können wie Zinndichloriddihydrat-HCl oder katalytische Reduktionen.

Schema 2

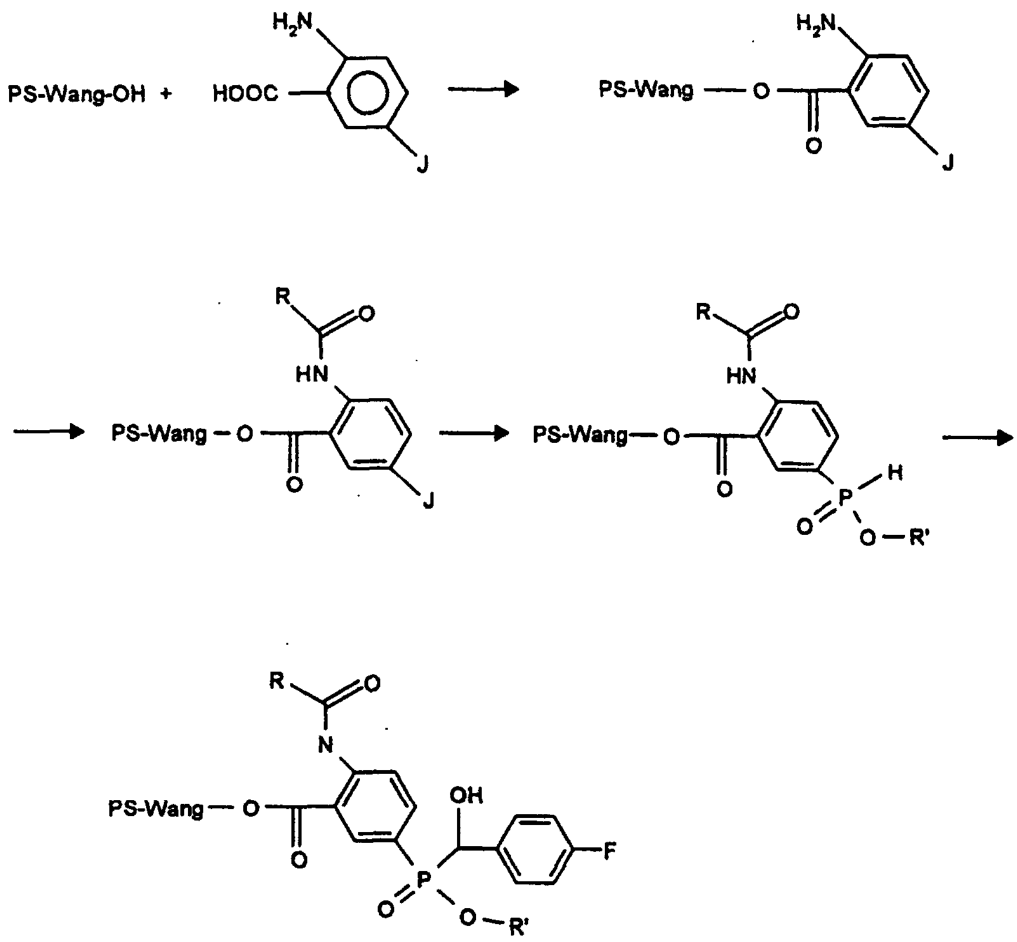

Die erhaltene Aminoverbindung läßt sich weiter modifizieren, z.B. durch (reduktive) Alkylierung oder wie hier gezeigt, durch Acylierung. Die Acylierung kann wiederum mit einer Vielzahl von Acylierungsmitteln erfolgreich durchgeführt werden, beispielsweise mit Carbonsäurehalogeniden oder Carbonsäuren ggf. unter Zusatz geeigneter aktivierender Reagentien wie beispielsweise Triethylamin oder Carbodiimiden. Anschließend wird das erfindungsgemäße Verfahren zum Aufbau der palladiumkatalysierten Darstellung der Phosphorkohlenstoffbindung eingesetzt.
Eine weitere Derivatisierung kann dann analog Schema 1 durchgeführt werden, z. B. die Reaktion mit 4-Fluor-benzaldehyd zur letzten Verbindung nach Schema 2.

Beispiele

Abkürzungen:

Die handelsübliche Polystyrol-Wang-Linker-Verbindung: (Fa. Rapp Polymere, Tübingen) 4-Hydroxymethylphenyloxymethyl-polystyrolharz, wird im folgenden als Hydroxy-Wangpolystyrolharz abgekürzt.

DMF = Dimethylformamid
THF = Tetrahydrofuran
TFA = Trifluoressigsäure
TMS = Trimethylsilan oder Trimethylsilyl
$CH_2Cl_2$ = Dichlormethan, Methylenchlorid
DBU = 1,8-Diazabicyclo[5.4.0]undec-7-en

DMSO = Dimethylsulfoxid
min = Minute(n)
h = Stunde(n)

% und andere Mengenverhältnisse beziehen sich auf das Gewicht, sofern sie im Text nicht speziell anders definiert sind.

1) Herstellung der Harz-Linker-Verbindung

4-Jodbenzoyl-oxy-Wangpolystyrolharz

500 ml wasserfreies Methylenchlorid wurden zusammen mit 12,5 g, (15,4 mmol Hydroxyfunktion) Hydroxy-Wangpolystyrolharz (200-400 mesh, 1,23 mmol OH pro Gramm Harz) vorgelegt. Zu dieser Suspension gab man 11,44 g (4,6 mmol) 4-Jodbenzoesäure, 0,83 g (6,55 mmol) Dimethylaminopyridin und 10,5 g (83,1 mmol) Diisopropylcarbodiimid. Die Suspension wurde 3 h geschüttelt und für 16 h stehen gelassen. Die Suspension wurde filtriert und das Harzpolymer mehrfach mit DMF (5-fach), THF (5-fach) und Methylenchlorid (5-fach), je insgesamt 1 l Lösungsmittel, gewaschen. Anschließend wurde mit 400 ml Diethylether nachgewaschen. Das Rohprodukt wurde im Vakuum getrocknet. Rohausbeute: 15,41 g (82,2 %).

2) Herstellung des Harz-Linker-Addukts

4-(Ethoxyphosphinoyl)-benzoyloxy-Wangpolystyrolharz (= Phosphonigsäureethylester (IVa))

15,00 g (14,4 mmol Jodarylfunktion) 4-Jodbenzoyloxy Wangpolystyrolharz wurden unter Argonatmosphäre in 250 ml Tetrahydrofuran suspendiert und mit 4,75 g (6,80 mmol) Bistriphenylphosphan-palladium-II-dichlorid und 17,46 g (172 mmol) wasserfreiem N-Methylmorpholin versetzt. Zu dieser Suspension wurde unter Schutzgasatmosphäre die wie folgt dargestellte Lösung gegeben: 10,44 g (158,2 mmol) wasserfreie unterphosphorige Säure, dargestellt durch Evaporation von 21 g einer 50 %igen wässrigen Lösung dieser Säure und anschließender einstündiger Trocknung über 4 Å Molsieb, wurden in 184,4 g Orthoameisensäuretriethylester (1,244 mol) 2h gerührt. Nach Zugabe wurde die Suspension sofort auf 65°C erhitzt und für 45 min unter Rückfluß gehalten. Anschließend kühlte man unter Schutzgas ab, filtrierte das Harzpolymer ab und wusch mit 400 ml einer 5 % igen Essigsäure in Tetrahydrofuran und anschließend mit 600 ml Tetrahydrofuran, mehrfach mit Methylenchlorid und mehrfach mit Diethylester. Das Harz wurde unter Vakuum getrocknet. Rohausbeute: 14,53 g (100,14 %)

3) P-(4-Carboxyphenyl)-P-[(4-Nitrophenyl)-hydroxymethyl]-phosphinsäure-ethylester

300 mg (0,3 mmol) 4-(Ethoxyphosphinoyl)-benzoyloxy-Wangpolystyrolharz wurden in 2 ml wasserfreiem Methylenchlorid suspendiert. Anschließend setzte man bei Kühlung auf 5°C. 7,50 ml einer 1 M Triethylaminlösung in Methylenchlorid und anschließend 6,75 ml einer 1 M Trimethylsilylchloridlösung in Methylenchlorid zu. Die Reaktionsmischung wurde innerhalb 1 h auf Raumtemperatur aufgewärmt, anschließend unter Schutzgas filtriert und dann mit 6,0 ml einer 1 M 4-Nitro-benzaldehydlösung in Methylenchlorid versetzt. Nach 2 h Schütteln bei Raumtemperatur filtrierte man das Harz ab und wusch mehrfach mit Tetrahydrofuran und Methylenchlorid nach. Das Harz wurde im Vakuum getrocknet. Anschließend spaltete man das Produkt ab, indem man das Harz für 1 h in einer 20 %igen Trifluoressigsäurelösung in Methylenchlorid schüttelte. Nach Filtration und Einengen der organischen Phase erhält man das Produkt ohne jegliche weitere Reinigung in über 90 %iger Reinheit. Ausbeute: 100 mg (92 % d.Th.)
$^1$H-NMR (TMS/DMSO $d_{6)}$:
$\delta$ (ppm) =1,08 (t, J = 6,9 Hz, 1,8 H, $CH_2$-$C\underline{H}_3$, Diastereomer 1), 1,10 (t, J = 6,9 Hz, 1,2 H, $CH_2$-$C\underline{H}_3$, Diastereomer 2), 3,88 (q, J = 6,9 Hz, 2,6 H, $C\underline{H}_2$-$CH_3$,
Diastereomer 1), 3,94 (q, J = 6,9 Hz, 1,4 H, $C\underline{H}_2$-$CH_3$, Diastereomer 2), 5,35 (d, J = 12 Hz, 0,6 H, C$\underline{H}$-OH, Diastereomer 1 ), 5,43 (d, J = 16 Hz, 0,4 H, C$\underline{H}$-OH, Diastereomer 2), 6,4 (s br., 1 H, COO$\underline{H}$), 7,48 - 7,60 (m, 3H, aromatische H, 7,72 - 7,83 (m, 3 H, aromatische H) 8,0 - 8,2 (m, 2 H, aromatische H), 8,13 - 8,2 (m, 1 H, aromatische H).

4) P-(4-Carboxyphenyl)-P-[N-isopropyl-1-aminoethyl]-phosphinsäureethyl-ester

1,00 g (1,0 mmol P-H-Funktionalität) 4-(Ethoxyphosphinoyl)-benzoyloxy-Wangpolystyrolharz wurden mit 2 ml Methylenchlorid vorgequollen, anschließend mit 2,30 g (22,7 mmol) Triethylamin in 2,0 ml Methylenchorid versetzt und dann mit einer Lösung von 2,24 g (20,7 mmol) Trimethylsilylchlorid umgesetzt. Nach 1,5 h Schütteln bei Raumtemperatur wurde die Flüssigkeit abfiltriert und eine Lösung von 2,55 g (29,9 mmol) N-Isopropyl-ethanimin in 4 ml Methylenchlorid zugegeben. Die Suspension wurde 3 h bei Raumtemperatur geschüttelt. Darauf wurde abfiltriert und

die Suspension mehrfach mit Methylenchlorid, Tetrahydrofuran und abschließend nochmals mit Methylenchlorid und Diethylether gewaschen.
Rohausbeute: 1,07 g (99 %)
Die Abspaltung von 27,3 mg des Rohproduktes mit einer 50 %igen Lösung von TFA in Dichlormethan führte zu 9,2 mg des Titelprodukts (94,6 % Ausbeute).
$^1$H-NMR (DMSO-$d_6$/TMS):
δ (ppm) = 1,05 - 1,45 (m, br., 12H, alle C$\underline{H}_3$), 3,42 (sept., J = 7,0 Hz, 0,6 H), $(CH_3)_2$-C$\underline{H}$ Diastereomer 1), 3,60 (sept, J = 7,0 Hz, 0,4 H, $(CH_3)_2$-C$\underline{H}$ Diastereomer 2), 3,90 bis 4,20 (m br., 3 H, $CH_2O$, P-C$\underline{H}$-N), 7,96 (m, 2H, aromatische H), 8,70 (m, 2H, aromatische H), 9,0 (s. br, 2H COOH, NH)

5) 4-(Hydroxyphosphinoyl)-benzoyloxy-Wangpolystyrolharz (= Phosphonigsäure (Va))

7,00 g (6,90 mmol P-H Funktionalität) 4-(Ethoxyphosphinoyl)-benzoyloxy-Wangpolystyrolharz wurden in 70 ml Tetrahydrofuran vorgelegt, mit 312 mg (17,3 mmol) Wasser und 5,28 g (34,7 mmol) DBU versetzt. Nach 1 h schütteln bei Raumtemperatur, filtrierte man die Suspension und wusch die feste Phase je fünfmal mit 100 ml 5 %iger Essigsäure in THF, mit 100 ml THF mit 100 ml Methylenchlorid und abschließend zweimal mit Diethylether. Das Harzpolymer trocknete man für 12 h im Vakuum. Rohausbeute: 7,57 g (112 %) der Titelverbindung

6) P-(4-Carboxyphenyl)-P-(1-isopropyl-1-hydroxy-methyl)-phosphinsäure

50 mg (0,05 mmol P-H-Funktionalität) 4-(Hydroxyphosphinoyl)-benzoyloxy-Wangpolystyrolharz wurden mit 1 ml Methylenchlorid versetzt. Nach 30 min wurden 1,1 ml einer 1 M Triethylaminlösung in Methylenchlorid zugegeben und anschließend mit 1 ml einer 1 M Trimethylsilylchloridlösung in Methylenchlorid versetzt. Nach 30 min Schütteln bei Raumtemperatur wurde die Flüssigkeit abfiltriert und mit 1 ml einer 1 M Lösung von Isobutyraldehyd in Methylenchlorid versetzt und 1 h bei Raumtemperatur geschüttelt. Darauf filtrierte man die Flüssigkeit ab und wusch das Harz je zehnfach mit THF und Methylenchlorid. Das Harz wurde anschließend für 30 min mit 5 ml 50 %iger Trifluoressigsäure in Methylenchlorid versetzt und filtriert. Das Filtrat wurde eingeengt.
Rohausbeute: 14 mg der Titelverbindung (108 %).
$^1$H-NMR (DMSO-$d_6$/TMS):
δ (ppm) = 0,95 (d, J = 11 Hz, 3 H, C$\underline{H}_3$-CH, Rotationsisomeres 1), 0,98 (d, J = 11 Hz, 3H, C$\underline{H}_3$-CH, Rotationsisomeres 2), 1,95 (sept, J = 11 Hz, 1H $(CH_3)_2$-CH-), 3,50 (t, J = 6 Hz, C$\underline{H}$-OH), 4,0 - 5,0 (s br., 3 H, COOH, PO$\underline{H}$ und CH-O$\underline{H}$), 7,8 (m, 2H, aromatische H), 8,0 (m, 2H, aromatische H)

7) P-(4-Carboxyphenyl)-P-(1-(4-Chlorphenyl)-1-hydroxy-methyl)-phosphinsäure

Das Verfahren folgte dem unter Beispiel 6 angegebenen Vorgehen, setzte aber als Aldehyd 4-Chlorbenzaldehyd ein. Rohausbeute: 17,3 mg Titelverbindung (105,8 %).
$^1$H-NMR (DMSO, TMS):
δ (ppm) = 4,0 (s, br, 3 H, COOH, P-O$\underline{H}$, C-O$\underline{H}$), 4,93 (d, J = 11,2 Hz, 1H, C$\underline{H}$-O), 7,24 (m, 4H, aromatische H), 7,72 (m, 2H, aromatische H), 7,98 (m, 2H, aromatische H).

8) P-(4-Carboxyphenyl)-P-(2-ethoxycarbonyl-ethyl)-phosphinsäure

50 mg (0,05 mmol OH-Funktionalität) 4-(Hydroxyphosphinoyl)-benzoyloxy-Wangpolystyrolharz wurden mit 1 ml Methylenchlorid versetzt und anschließend mit 392 mg (19 mmol) Bistrimethylsilylacetamid, gelöst in 2 ml Methylenchlorid, bei Raumtemperatur für 1 h zur Reaktion gebracht. Die Flüssigkeit wurde abfiltriert und die oben beschriebene Umsetzung wiederholt. Das filtrierte Harz wurde dann mit 1 ml einer 1 M Lösung von Acrylsäureethylester in Methylenchlorid versetzt und für 16 h bei Raumtemperatur stehengelassen. Die Flüssigkeit wurde abfiltriert und das Harz je zehnfach mit THF und Methylenchlorid gewaschen. Das Harz wurde dann 30 min mit einer 50 %igen Lösung von Trifluoressigsäure in Methylenchlorid umgesetzt. Das Filtrat ergab nach Einengen das Rohprodukt in über 90 %iger Reinheit; Ausbeute: 17,5 mg (122 %).
$^1$H-NMR (DMSO-$d_6$ / TMS):
δ (ppm) = 1,12 (t, 7,2 Hz, 3H, $CH_2$-$CH_3$), 2,04 (m, 2H, C$\underline{H}_2$-COOEt), 2,38 (m, 2H, $CH_2$-$CH_2$COOEt), 3,98 (q, J = 7,2 Hz, OC$\underline{H}_2$-$CH_3$), 7,82 (m, 2H, aromatische H), 8,06 (m, 2H, aromatische H).

9) P-(4-Carboxyphenyl)-P-[N-benzyl-1-isopropyl-amino-methyl]-phosphinsäure

Das Verfahren folgte exakt dem unter Beispiel 8 angegebenen Vorgehen. Anstelle der Aldehydlösung wurde

15 ml einer 0,5 molaren Lösung von N-Benzyl-isopropylmethanimin in Methylenchlorid zugegeben und 3 h bei Raumtemperatur gerührt. Die Flüssigkeit wurde abfiltriert und das Harz je zehnfach mit Methylenchlorid, THF und wieder Methylenchlorid gewaschen. Die Abspaltung erfolgte durch 30 minütige Umsetzung des Harzes mit 3 ml einer 50 %igen Trifluoressigsäurelösung in Methylenchlorid.
Nach Einengen der Lösung erhielt man das Rohprodukt in über 90 %iger Reinheit. Rohausbeute: 22 mg (126 %)
$^1$H-NMR (DMSO-$d_6$ / TMS):
δ = 0,82 (d, J = 8,0 Hz, 3H, C<u>H</u>$_3$-CH-$CH_3$), 0,98 (d, J = 7,0 Hz, 3H, $CH_3$-CH-$CH_3$), 2,10 (m, 1H, $(CH_3)_2$C<u>H</u>), 3,06 (dd, $J_{PH}$ = 12,4 Hz, $J_{C-H}$ = 4 Hz, 1H, P-C<u>H</u>-C), 4,34 (AB-Spektrum, J<u>HAHB</u> = 12,0 Hz, 2H, C<u>H</u>$_2$-N), 7,4 (m, 5H, aromatische H), 7,82 (m, 2H, aromatische H), 8,06 (m, 2H, aromatische H).

10) 2-Amino-5-iodbenzoyloxy-Wangpolystyrolharz

15,0 g (18,5 mmol) Hydroxy-Wangpolystyrolharz (200 - 400 µm, Fa. Rapp Polymere) wurden unter Schutzgasatmosphäre in 200 ml wasserfreiem Methylenchlorid suspendiert. Anschließend gab man 14,56 g (55 mmol) Jodanthranilsäure, 12,58 g (99,6 mmol) Diisopropylcarbodiimid und 0,990 g (8,10 mmol) 4-N,N-Dimethylaminopyridin zu und schüttelte die Suspension für 24 h. Das Harz wurde abfiltriert und fünffach mit insgesamt 2 l DMF, fünffach mit insgesamt 1 l THF und mehrfach mit insgesamt 1 l Methylenchlorid gewaschen. Der Feststoff wurde dann zweimal mit Ether gewaschen und dann im Vakuum gründlich getrocknet. Ausbeute: 18,58 g (94,9 %).

11) 2-Isopropylcarbonylamino-5-iodbenzoyloxy-Wangpolystyrolharz

550 mg (0,52 mmol) 2-Amino-5-iodbenzoyloxy-Wangpolystyrolharz wurde in 15 ml wasserfreiem Methylenchlorid suspendiert, auf 0°C gekühlt und anschließend mit 526 mg (5,2 mmol) Triethylamin und 554 mg Isobuttersäurechlorid versetzt. Die Reaktionslösung erwärmte sich auf Raumtemperatur. Nach 16 h Rühren. Darauf filtrierte man die feste Phase ab und wusch fünfzehnfach mit Methylenchlorid und dreifach mit Ether nach. Ausbeute: 530 mg = 90,4 %

12) 5-Ethoxyphosphinoyl-2-isopropylcarbonylamino-benzoyloxy-Wangpolystyrolharz

0,500 g (0,443 mmol Aryl-iod-Funktionalität) 2-Isopropylcarbonylamino-5-iodbenzoyloxy-Wangpolystyrolharz wurden in 5 ml wasserfreiem THF unter Argonatmosphäre suspendiert und mit 0,538 g (5,30 mmmol) N-Methylmorpholin und 0,146 g (0,208 mmol) Bistriphenylphosphan-palladium-II-dichlorid versetzt. Anschließend wurde zu dieser Suspension eine wie folgt dargestellte Lösung gegeben:
0,320 g (4,90 mmol) kristalline, unterphosphoriger Säure wurden mit 4 Å Molsieb unter Schutzgasatmosphäre 1 h getrocknet und mit 5,68 g (38,3 mmol) Triethylorthoformiat 2,5 h gerührt. Diese Lösung wurde zur oben beschriebenen Suspension gegeben, die darauf schnell für 1 h auf Rückflußtemperatur erwärmt wurde. Der Ansatz wurde danach schnell abgekühlt, zehnfach mit einer 5 %igen Essigsäurelösung in Tetrahydrofuran gewaschen, zehnfach mit Methylenchlorid gewaschen und abschließend dreimal mit Diethylether nachgewaschen. Das entstandene Harz wurde im Vakuum getrocknet; Rohausbeute: 458 mg (94,5 %). Eine Probeabspaltung mit 50 mg Harz (1 h 20 %ige Trifluoressigsäure/Methylenchlorid) ergab 17,3 mg (105 %) des Rohprodukts.
$^1$H-NMR (DMSO-$d_6$/TMS) :
δ (ppm) = 1,08 (d, J = 8,0 Hz, 6H, (C<u>H</u>$_3$)$_2$CH), 1,23 (t, J = 5,6 Hz, 3H, $CH_2$-C<u>H</u>$_3$), 2,60 (sept, J = 8,0 Hz, 1H, ($CH_3$)C<u>H</u>), 4,07 (m, 2H, P-O-C<u>H</u>$_2$), 7,55 (d, J = 576 Hz, 1H, P-H), 7,83 (m, 1H, aromatische H), 8,35 (m, 1H, aromatische H), 8,73 (m, 1H, aromatische H).

13) P-(3-Carboxy-4-isopropylcarbonylaminophenyl)-P-(pyrid-4-yl-hydroxymethyl)-phosphinsäureethylester

50 mg (0,046 mmol P-H-Funktionalität)5-Ethoxyphosphinoyl-2-isopropylcarbonylamino-benzoyloxy-Wangpolystyrolharz wurden in 1 ml Methylenchlorid suspendiert, mit 1,1 ml einer 1 M Triethylaminlösung in Methylenchlorid und 1,0 ml eine 1 M Trimethylsilylchloridlösung in Methylenchlorid versetzt. Nach 30 min Schütteln wurde die Lösung abfiltriert und mit 1 ml einer 1 M Lösung von 4-Nicotinaldehyd in Methylenchlorid versetzt. Nach 1 h Reaktion bei Raumtemperatur filtrierte man die Reaktionslösung ab, das Harz zehnfach mit Tetrahydrofuran und Methylenchlorid nach und setzte das Produkt vom Harz frei, indem man für 30 min mit 50 %iger Trifluoressigsäure in Methylenchlorid abspaltete. Das Rohprodukt wurde am Rotationsverdampfer eingeengt und in einer Reinheit von über 95 % als glasartige Masse erhalten. Ausbeute: 21 mg (96 %).
$^1$H-NMR (DMSO-$d_6$/TMS):
δ (ppm) = 1,13 (t, 6,4 Hz, 2H, O-$CH_2$-C<u>H</u>$_3$ Diastereomer 1), 1,18 (d, 7 Hz, 6H, (C<u>H</u>$_3$)$_2$CH), 1,23 (t, 6,4 Hz, 1,8 H, $OCH_2CH_3$, Diastereomer 2), 2,60 (sept, J = 7 Hz, 1H, $(CH_3)_2$-CH), 3,89 (quart, J = 8,0 Hz, 0,8 H, C<u>H</u>$_2$-$CH_3$, Diastereomer

1), 4,06 (quart, J = 8,0 $H_2$, 1,2 H, $CH_2$-$CH_3$, Diastereomer 2), 5,43 (d, J = 12,8 Hz, 0,4 H, P-C$\underline{H}$ Diastereomer 1), 5,60 (d, J = 16,0 Hz, 0,6 H, P-C$\underline{H}$ Diastereomer 2), 7,97 (m, 2H, aromatische H, 7,80 (m, 1H, aromatische H), 8,28 (m, 1H, aromatisches H), 8,64 (m, 1H, aromatische H), 8,76 (m, 2H, aromatische H), 11,4 (s, 1H, COOH).

14) N-(4-Iod)benzoylamino)-Rink-Amid-polystyrolharz

10,00 g (7,8 mmol NH-Funktionalität) Fmoc-Rink-Amid-polystyrolharz wurden von der Fmoc-Gruppe entschützt, indem man mit 100 ml einer 20 %igen Piperidin/DMF-Lösung 30 min schüttelte und anschließend abfiltrierte. Das Verfahren wurde einmal wiederholt und das Harz danach gründlich mit DMF gewaschen. Das Harz wurde erneut in 120 ml DMF suspendiert und mit 6,15 g (25,0 mmol) 4-Iodbenzoesäure, 3,20 g (25,0 mmol) Diisopropylcarbodiimid und 3,33 g (25,0 mmol) 1-Hydroxybenztriazol versetzt. Nach 4 stündigem Schütteln wurde das Harz abfiltriert und anschließend je fünfmal mit DMF, THF und Methylenchlorid gewaschen. Das Harz wurde im Vakuum getrocknet. Rohausbeute: 9,80 g (99 % d.Th.) der Titelverbindung.

15) N-[4-(Ethoxyphosphinoyl)-benzoyl]-Rink-Amid-polystyrolharz

1,00 g (0,77 mmol Iodarylfunktion) N-(4-Iodbenzoyl)-Rink-Amid-polystyrolharz wurden unter Argonatmosphäre in 10 ml wasserfreiem Tetrahydrofuran suspendiert und mit 258 mg (0,370 mmol) Bistriphenylphosphanpalladium-II-dichlorid und 1,00 ml (9,09 mmol, 0,92 g) wasserfreiem N-Methylmorpholin versetzt. In diese Suspension wurde unter Schutzgasatmosphäre die wie folgt dargestellte Lösung gegeben:
0,55 g (8,3 mmol) wasserfreie unterphosphorige Säure, dargestellt durch Evaporation von 1,1 g einer 50 %igen wässrigen Lösung dieser Säure und einstündiger Trocknung über 4 Å Molsieb und 10,0 g (67,5 mmol) Triethylorthoformiat wurden 2 h zusammen gerührt.
Nach Zugabe der erhaltenen Lösung wurde die Suspension sofort auf 65°C erhitzt und für 45 min. unter Rückfluß gehalten. Anschließend kühlte man unter Schutzgas ab, filtrierte das Harzpolymer ab und wusch mit 50 ml einer 5 %igen Essigsäure in Tetrahydrofuran und anschließend mit 150 ml Tetrahydrofuran, mehrfach mit Methylenchlorid und mehrfach mit Diethylether. Das Harz wurde unter Vakuum getrocknet. Ausbeute: 0,950 g (97 % d. Th.) der Titelverbindung.

16) P-(4-Aminocarbonylphenyl)-P-[(4-Fluorphenyl)-hydroxymethyl]phosphinsäure-ethylester

50 mg (0,063 mmol P-H-Funktionalität) N-[4-(Ethoxyphosphinoyl)-benzoyl]-RinkAmid-Polystyrolharz wurden mit 1,5 ml (1,5 mmol) einer 1 M Lösung von 1,8-Diazabicyclo[5.4.0]undec-7-en (DBU) in Dichlormethan versetzt. Nach 15 min. wurde die Lösung abfiltriert und mit 1,5 ml einer 1 M Lösung von 4-Fluorbenzaldehyd in Dichlormethan versetzt. Nach 1 h Schütteln bei Raumtemperatur filtrierte man das Harz ab und wusch mehrfach mit Tetrahydrofuran und Dichlormethan nach. Das Harz wurde im Vakuum getrocknet. Anschließend spaltete man das Produkt ab, indem man das Harz für 1 h in einer 20 %igen Trifluoressigsäurelösung in Dichlormethan schüttelte. Nach Filtration und Einengen der organischen Phase erhielt man das Produkt ohne jegliche weitere Reinigung in über 90 %iger Reinheit. Ausbeute: 11 mg (82 % d. Th.).
$^1$H-NMR (DMSO-$d_6$/TMS):
δ (ppm) = 1,18 (t, J = 6,9 Hz, 3H, $CH_2$-C$\underline{H}_3$, Diastereomer 1), 1,19 (t, J = 6,9 Hz, 3H, $CH_2$-C$\underline{H}_3$, Diastereomer 2), 3,85 (q, J = 6,9 Hz, 2H, $\underline{CH_2}$-$CH_3$, Diastereomer 1), 3,91 (q, J = 6,9 Hz, 2H, $\underline{CH_2}$-$CH_3$, Diastereomer 2), 5,10 (d, J = 12 Hz, 1H, C$\underline{H}$-OH,
Diastereomer 1), 5,22 (d, J = 15 Hz, 1H, C$\underline{H}$-OH, Diastereomer 2), 7,10 (t, J = 6 Hz, 2H, aromatische H), 7,31 (m, 2H, aromatische H), 7,55 (s, br, 1H, N$\underline{H}$), 7,7 (m, 2H, aromatische H), 7,91 (m, 2H, aromatische H), 8,12 (s, br, 1H, N$\underline{H}$).

17) Aufbau einer Substanzbibliothek gemäß Schema 1 und Schema 2

Die im obengenannten Text dargestellte experimentelle Vorgehensweise erlaubt den systematischen Aufbau von Substanzbibliotheken. Ausgehend von 5-Iodanthranilsäure, das durch den Wang-Linker über die Carbonsäurefunktion an ein Polymer gebunden ist, wurde durch folgende schrittweise Umsetzungen eine Substanzbibliothek aufgebaut:
Die Verbindung der Formel

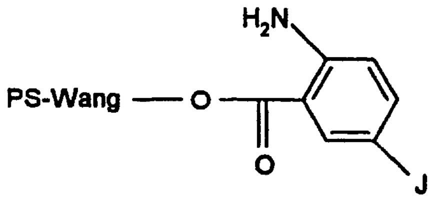

wurde mit den Acylierungsmitteln Acetylchlorid, Propionylchlorid, Isopropylcarbonylchlorid und Cyclohexylcarbonylchlorid zu den vier verschiedenen N-Acylverbindungen umgesetzt. Die palladiumkatalysierte Umsetzung mit Bis-triphenylphosphan-Pd-II-dichlorid mit unterphosphoriger Säure und Orthoameisensäuretriethylester analog Beispiel 2 führte zu den vier Phosphonigsäureethylestern gemäß der Formel (IV) und anschließende Hydrolyse zu den vier Phosphonigsäuren gemäß der Formel (V). Die acht erhaltenen phosphorhaltigen Harz-Linker-Addukte gemäß (IV) und (V) wurden jeweils mit 10 unterschiedlichen Aldehyden und 10 unterschiedlichen Isocyanaten gemäß Tabellen A und B umgesetzt:

Tabelle A:

| Aldehyde der Formel (A1) | |
|---|---|
| H-CO-R* (A1) | |
| Nr. | R* |
| 1 | Phenyl |
| 2 | 4-Chlor-phenyl |
| 3 | 4-Methoxy-phenyl |
| 4 | 3,4-Dichlor-phenyl |
| 5 | 2-Chlor-phenyl |
| 6 | 2,4-Dichlor-phenyl |
| 7 | 2-Fluor-phenyl |
| 8 | 3-Brom-4-fluor-phenyl |
| 9 | 2-Methoxy-phenyl |
| 10 | 2,6-Dichlor-phenyl |

Tabelle B:

| Isocyanate der Formel (A2) | |
|---|---|
| O=C=N-R* (A2) | |
| Nr. | R* |
| 1 | 4-Chlor-phenyl |
| 2 | 3,4-Dichlor-phenyl |
| 3 | 3,5-Dichlor-phenyl |
| 4 | 2,4-Dichlor-phenyl |
| 5 | 4-Brom-phenyl |
| 6 | 4-Isopropyl-phenyl |
| 7 | 1-Naphthyl |
| 8 | 3-(2,2-Dichlor-1,1-difluorethoxy)-phenyl |
| 9 | 3-Methoxycarbonyl-phenyl |
| 10 | 2-Butoxy-phenyl |

Die erhaltene Substanzbibliothek aus 160 Harz-Linker-Addukten gemäß der allgemeinen Formel (IX)

$$[\text{ Harzpolymer }] - [\text{ Linker - Z - } R^1 - P(R^2)(=O) - O - R^3 ]_n \qquad (IX),$$

in der $R^2$ ein Rest der Formeln ($R^2a$) bzw. ($R^2b$) ist,

-CHOH-R* ($R^2a$)

-CO-NH-R* ($R^2b$)

worin R* gemäß Tabellen A bzw. B definiert ist, und die übrigen Reste und Gruppen wie vorstehend definiert sind, wurden mit 20%iger Trifluoressigsäure in Methylenchlorid gespalten und eine weitere Substanzbibliothek aus 160 Verbindungen der Formel (Ib) erhalten,

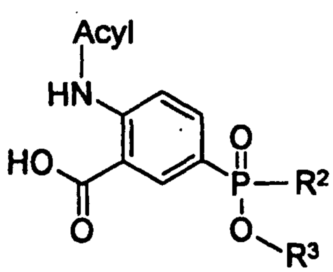

(Ib)

worin

Acyl = Acetyl, Propionyl, Isopropylcarbonyl oder Cyclohexylcarbonyl,
$R^2$ = ($R^2a$) oder ($R^2b$), welche die genannten 20 verschiedenen Reste sind,
$R^3$ = H oder Ethyl und
Y = COOH

bedeuten.

## Patentansprüche

**1.** Verfahren unter Verwendung von Zwischenprodukten, die an ein Harzpolymer gebunden sind, zur Herstellung von chemischen Verbindungen der Formel (I),

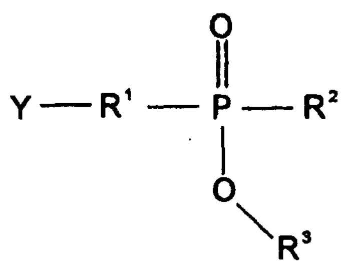

(I)

worin

$R^1$ einen unsubstituierten oder substituierten aromatischen oder heteroaromatischen Rest bedeutet,
$R^2$ Wasserstoff oder einen organischen Rest bedeutet, der auch über Heteroatome mit dem restlichen Teil der Verbindung der Formel (I) verbunden sein kann,
$R^3$ Wasserstoff oder einen über ein C-Atom gebundenen organischen Rest bedeutet und
Y die funktionelle Gruppe bedeutet, die nach Abspaltung der Verbindung (I) vom Harzpolymer am Molekül der Formel (I) gebildet wird,

**dadurch gekennzeichnet, daß** man

a) ein Harz-Linker-Addukt der Formel (II)

$$[\text{ Harzpolymer }]\text{-}[\text{ Linker - Z - }E^1\text{ - }S^1\text{ }]_n \qquad \text{(II)}$$

worin

| | |
|---|---|
| [ Harzpolymer ] | für den Rest eines Harzes steht, der in der Harz-Linker-Verbindung (II) über n Bindungsstellen mit den n Gruppen der Formel [ Linker-Z-$E^1$-$S^1$] verbunden ist, |
| Linker | jeweils einen organischen Linker bedeutet, |
| Z | eine für den jeweiligen Linker spezifische funktionelle Gruppe oder Bindung bedeutet, aus der nach Abspaltung der Verbindung (I) vom Harzpolymer-Linker-Rest die Gruppe Y in Formel (I) entsteht, |
| $E^1$ | wie $R^1$ in Formel (I) definiert ist oder einen für die Herstellung von $R^1$ in Verbindung (I) geeigneten Rest bedeutet, |
| $S^1$ | eine funktionelle Gruppe ist, die für palladiumkatalysierte Substitutionen analog der Heck-Reaktion geeignet ist, |
| n | die Anzahl der funktionellen Gruppen [Linker-Z-$E^1$-$S^1$] am Harz bedeutet, die vom Molekulargewicht des Harzpolymers abhängt und größer oder gleich 1 ist, |

in Gegenwart eines geeigneten Palladiumkatalysators mit einer Verbindung aus der Gruppe der Phosphinate (Derivate der unterphosphorigen Säure) der Formel (III)

$$A^1\text{-O-(PHO)}A^* \qquad \text{(III)}$$

worin

| | |
|---|---|
| $A^1$ | Wasserstoff oder einen organischen Rest bedeutet, |
| $A^*$ | eine hydrolytisch abtrennbare oder nach einer Zwischenreaktion abtrennbare Gruppe ist, |

unter Substitution der Gruppe $S^1$ zu einer harzgebundenen Verbindung der Formel (IV) umsetzt,

$$[\text{ Harzpolymer }]\text{ - }[\text{ Linker - Z - }E^1\text{ - P(H)(=O) - O - }A^1\text{ }]_n \qquad \text{(IV)}$$

worin $A^1$ wie in Formel (III) definiert ist, und

b) gegebenenfalls die Verbindung (IV) in einem oder mehreren weiteren Reaktionsschritten am organischen Rest $E^1$ zum Rest $(E^1)'$ derivatisiert und damit eine bzw. mehrere harzgebundene Zwischenstufen der Formel (IV)' erhält,

$$[\text{ Harzpolymer }]\text{-}[\text{ Linker - Z - }(E^1)'\text{ - P(H)(=O) - O - }A^1\text{ }]_n \qquad \text{(IV)'}$$

worin $A^1$ wie in Formel (III) definiert ist, und

c) gegebenenfalls die Verbindung der Formel (IV) bzw. (IV)' aus Stufe a) bzw. b) zu einer für die harzgebundene Synthese geeigneten Verbindung (V) bzw. (V)' hydrolysiert,

$$[\text{ Harzpolymer }]\text{ - }[\text{ Linker - Z - }E^1\text{ - P(H)(=O) - OH }]_n \qquad \text{(V)}$$

$$[\text{ Harzpolymer }]\text{-}[\text{ Linker - Z - }(E^1)'\text{ - P(H)(=O) - OH }]_n \qquad \text{(V)'}$$

und

d) gegebenenfalls die nach c) erhaltene Verbindung (V) bzw. (V)' zur Verbindung der Formel (VI) bzw. (VI)' verestert,

$$[\text{ Harzpolymer }] - [\text{ Linker - Z - }E^1\text{ - P(H)(=O) - O - }R^3]_n \qquad \text{(VI)}$$

$$[\text{ Harzpolymer }] - [\text{ Linker - Z - }(E^1)'\text{ - P(H)(=O) - O - }R^3]_n \qquad \text{(VI)'}$$

worin

$R^3$ wie $R^3$ in Formel (I) definiert, aber nicht gleich Wasserstoff ist, und

e) gegebenenfalls eine nach a), b), c) oder d) erhaltene Verbindung (IV), (V) oder (VI) bzw. (IV)', (V)' bzw. (VI) ', deren gemeinsames Strukturmerkmal die Phosphonigsäure- oder Phosphonigsäureester-gruppe ist, unter Ausbildung einer Phosphor-Kohlenstoffbindung zu Verbindungen der allgemeinen Formeln (VII) oder (VIII) bzw. (VII)' bzw. (VIII)' umsetzt,

$$[\text{ Harzpolymer }] - [\text{ Linker - Z - }E^1\text{ - P(}R^2\text{)(=O) - O - }A^4]_n \qquad \text{(VII)}$$

$$[\text{ Harzpolymer }] - [\text{ Linker - Z - }(E^1)'\text{ - P(}R^2\text{)(=O) - O - }A^4]_n \qquad \text{(VII)'}$$

$$[\text{ Harzpolymer }] - [\text{ Linker - Z - }E^1\text{ - P(}E^2\text{)(=O) - O - }A^4]_n \qquad \text{(VIII)}$$

$$[\text{ Harzpolymer }] - [\text{ Linker - Z - }(E^1)'\text{ - P(}E^2\text{)(=O) - O - }A^4]_n \qquad \text{(VIII)'}$$

worin

$R^2$ die in Formel (I) festgelegte Bedeutung hat,
$E^2$ xeinen organischen Rest bedeutet, der zu dem Rest $R^2$ derivatisiert werden kann,
$A^4$ = $A^1$, H bzw. $R^3$ bedeutet, und

f) die nach vorstehenden Stufen erhaltenen Verbindungen, falls erforderlich, an den Resten $E^1$, $(E^1)'$, $E^2$ und $A^4$ so modifiziert, daß die harzgebundene Verbindung der Formel (IX) erhalten wird,

$$[\text{ Harzpolymer }] - [\text{ Linker - Z - }R^1\text{ - P(}R^2\text{)(=O) - O - }R^3]_n \qquad \text{(IX)}$$

worin $R^1$, $R^2$, $R^3$ wie in Formel (I) definiert sind, und

g) die Verbindung der Formel (I), aus dem Harz-Linker Addukt der Formel (IX) abspaltet,

wobei in den Formeln (IV) bis (IX) und (IV)' bis (VIII)' die Reste [Harzpolymer], Linker, Z wie in Formel (II) definiert sind und $E^1$ bzw. $(E^1)'$ in den Formeln (V) bis (VIII) bzw. (V)' bis (VIII)' wie in Formel (IV) bzw. (IV)' definiert sind.

2. Verfahren nach Anspruch 1, **dadurch gekennzeichnet, daß**

$R^1$ Phenylen bedeutet, das unsubstituiert oder durch einen bis vier Reste aus der Gruppe Halogen, Alkyl, Haloalkyl, Alkoxy, Haloalkoxy, Alkylthio, Hydroxy, Amino, Nitro, Cyano, Azido, Alkoxycarbonyl, Alkylcarbonyl, Formyl, Carbamoyl, Mono- und Dialkylaminocarbonyl, Acylamino, Mono- und Dialkylamino, Alkylsulfinyl,

Haloalkylsulfinyl, Alkylsulfonyl und Haloalkylsulfonyl substituiert ist, wobei jeder Substituent bis zu 6 C-Atome im Alkylteil haben kann,
oder einen heteroaromatischen Rest aus der Gruppe der Fünf- oder Sechsringe mit jeweils 1, 2 oder 3 Heteroatomen aus der Gruppe N, O und S bedeutet, wobei der Rest unsubstituiert oder durch einen bis vier Reste aus der Gruppe Halogen, Alkyl, Haloalkyl, Alkoxy, Haloalkoxy, Alkylthio, Hydroxy, Amino, Nitro, Cyano, Azido, Alkoxycarbonyl, Alkylcarbonyl, Formyl, Carbamoyl, Mono- und Dialkylaminocarbonyl, substituiertes Amino wie Acylamino, Mono- und Dialkylamino, Alkylsulfinyl, Haloalkylsulfinyl, Alkylsulfonyl und Haloalkylsulfonyl substituiert ist und jeder Substituent bis zu 6 C-Atome im Alkylteil haben kann, und

$R^2$ Wasserstoff, einen aliphatischen Kohlenwasserstoffrest der unsubstituiert oder substituiert ist und inklusive Substituenten 1 bis 30 C-Atome enthält, bedeutet,

$R^3$ Wasserstoff oder einen aliphatischen Kohlenwasserstoffrest, der unsubstituiert oder substituiert ist und inklusive Substituenten 1 bis 30 C-Atome enthält, bedeutet oder einen Aryl oder Heteroarylrest, der unsubstituiert oder substituiert ist und inklusive Substituenten 1 bis 30 C-Atome enthält, bedeutet und

Y H, COOH, $CONH_2$, OH, $NH_2$ oder Alkylamino bedeutet.

3. Verfahren nach Anspruch 1 oder 2, **dadurch gekennzeichnet, daß**

$R^2$ Wasserstoff oder einen aliphatischen acyclischen oder cyclischen Kohlenwasserstoffrest mit 1 bis 20 C-Atomen oder Heterocyclyl mit 3 bis 7 Ringatomen und 1, 2 oder 3 Heteroatomen aus der Gruppe N, O und S bedeutet, wobei der Kohlenwasserstoffrest oder der Heterocyclylrest jeweils unsubstituiert oder durch einen oder mehrere Reste aus der Gruppe Halogen, Alkoxy, Alkenyloxy, Alkinyloxy, Haloalkoxy, Haloalkenyloxy, Haloalkinyloxy, Alkylthio, Amino, Nitro, Cyano, Azido, Alkoxycarbonyl, Alkenyloxycarbonyl, Alkinyloxycarbonyl, Alkylcarbonyl, Alkenylcarbonyl, Alkinylcarbonyl, Formyl, Carbamoyl, Mono- und Dialkylaminocarbonyl, Acylamino, Mono- und Dialkylamino, Alkylsulfinyl, Haloalkylsulfinyl, Alkylsulfonyl, Haloalkylsulfonyl, unsubstituiertes und substituertes Cycloalkyl, unsubstituiertes und substituertes Cycloalkenyl, unsubstituiertes und substituiertes Aryl, unsubstituiertes und substituiertes Heterocyclyl, unsubstituiertes und substituiertes Cycloalkoxy, unsubstituiertes und substituiertes Cycloalkenyloxy, unsubstituiertes und substituiertes Aryloxy, unsubstituiertes und substituiertes Heterocyclyloxy, unsubstituiertes und substituiertes Cycloalkylamino, unsubstituiertes und substituiertes Cycloalkenylamino, unsubstituiertes und substituiertes Arylamino, unsubstituiertes und substituiertes Heterocyclylamino, und im Falle cyclischer Reste auch Alkyl und Haloalkyl substituiert ist.

4. Verfahren nach Anspruch 3, **dadurch gekennzeichnet, daß**

$R^2$ ein Rest der Formel ($R^2$a), ($R^2$b), ($R^2$c), ($R^2$d) oder ($R^2$e) bedeutet,

-CHOH-R* ($R^2$a)

-CO-NH-R* ($R^2$b)

-CHR**-NH-R* ($R^2$c)

$-CR^aR^b-CR^cR^d-X-R^e$ ($R^2$d)

-R* ($R^2$e)

worin

X die elektronegative funktionelle Gruppe eines Michaelakzeptors,

R* einen aliphatischen acyclischen oder cyclischen Kohlenwasserstoffrest mit 1 bis 12 C-Atomen oder Heterocyclyl mit 3 bis 6 Ringatomen und 1, 2 oder 3 Heteroatomen aus der Gruppe N, O und S bedeutet, wobei der Kohlenwasserstoffrest oder der Heterocyclylrest jeweils unsubstituiert

oder durch einen oder mehrere Reste aus der Gruppe Halogen, Alkoxy, Alkenyloxy, Alkinyloxy, Haloalkoxy, Haloalkenyloxy, Haloalkinyloxy, Alkylthio, Amino, Nitro, Cyano, Azido, Alkoxycarbonyl, Alkenyloxycarbonyl, Alkinyloxycarbonyl, Alkylcarbonyl, Alkenylcarbonyl, Alkinylcarbonyl, Formyl, Carbamoyl, Mono- und Dialkylaminocarbonyl, Acylamino, Mono- und Dialkylamino, Alkylsulfinyl, Haloalkylsulfinyl, Alkylsulfonyl, Haloalkylsulfonyl, unsubstituiertes und substituiertes Cycloalkyl, unsubstituiertes und substituertes Cycloalkenyl, unsubstituiertes und substituiertes Aryl, unsubstituiertes und substituiertes Heterocyclyl, unsubstituiertes und substituiertes Cycloalkoxy, unsubstituiertes und substituertes Cycloalkenyloxy, unsubstituiertes und substituiertes Aryloxy, unsubstituiertes und substituiertes Heterocyclyloxy, unsubstituiertes und substituiertes Cycloalkylamino, unsubstituiertes und substituiertes Cycloalkenylamino, unsubstituiertes und substituiertes Arylamino, unsubstituiertes oder substituiertes Heterocyclylamino und im Falle cyclischer Reste auch Alkyl und Haloalkyl substituiert ist,

| | |
|---|---|
| $R^{**}$ | ein Rest aus der Gruppe der für $R^*$ definierten Reste ist oder |
| $R^*$ und $R^{**}$ | gemeinsam eine Alkylenbrücke bedeuten, welche unsubstituiert oder durch einen oder mehrere Reste substituiert ist, die unabhängig voneinander aus der Gruppe der Substituenten am Kohlenwasserstoffrest für $R^*$ ausgewählt sind, und |
| $R^a$, $R^b$, $R^c$, $R^d$, $R^e$ | unabhängig voneinander jeweils einen Rest aus der Gruppe der für $R^*$ definierten Reste ist oder |
| $R^a$, $R^c$ oder $R^d$, $R^e$ oder $R^c$, $R^e$ | paaweise eine Alkylenbrücke bedeuten, welche unsubstituiert oder durch einen oder mehrere Reste substituiert ist, die unabhängig voneinander aus der Gruppe der Substituenten am Kohlenwasserstoffrest für $R^*$ ausgewählt sind. |

**5.** Verfahren nach einem der Ansprüche 1 bis 4, **dadurch gekennzeichnet, daß** $R^3 = (C_1\text{-}C_4)$Alkyl und Y = COOH bedeuten.

**6.** Verfahren nach einem der Ansprüche 1 bis 5, **dadurch gekennzeichnet, daß**

| | |
|---|---|
| Z | eine Gruppe der Formel -O-CO- oder -NH-CO- ist und |
| $S^1$ | ein Jodatom bedeutet. |

**7.** Verfahren zur Herstellung einer Verbindung der Formel (IV)

$$[\text{ Harzpolymer }] - [\text{ Linker - Z - } E^1 \text{ - P(H)(=O) - O - } A^1 ]_n \qquad \text{(IV)}$$

worin $A^1$, [Harzpolymer], Linker, Z, $E^1$ und n wie in Anspruch 1 definiert sind und **dadurch gekennzeichnet, daß** man ein Harz-Linker-Addukt der Formel (II)

$$[\text{ Harzpolymer }] - [\text{ Linker - Z - } E^1 \text{ - } S^1 ]_n \qquad \text{(II)}$$

worin
[Harzpolymer], Linker, Z, $E^1$ und n wie in Formel (IV) definiert sind und $S^1$ eine funktionelle Gruppe ist, die für palladium-katalysierte Substitutionen analog der Heck-Reaktion geeignet ist,
in Gegenwart eines geeigneten Palladiumkatalysators mit einer Verbindung aus der Gruppe der Phosphinate (Derivate der unterphosphorigen Säure) der Formel (III)

$$A^1\text{-O-(PHO)}A^* \qquad \text{(III)}$$

worin

A1 Wasserstoff oder einen organischen Rest bedeutet,
A* eine hydrolytisch abtrennbare oder nach einer Zwischenreaktion abtrennbare Gruppe ist,

unter Substitution der Gruppe $S^1$ zur Verbindung der Formel (IV) umsetzt.

**8.** Verfahren zur Herstellung einer Verbindung der Formel (VII)

$$[\text{ Harzpolymer }] - [\text{ Linker - Z - } E^1 \text{ - } P(E^2)(=O) \text{ - O - } A^4 ]_n \quad (VII)$$

worin $A^4$, [Harzpolymer], Linker, Z, $E^1$, $E^2$ und n wie in Anspruch 1 definiert sind, **dadurch gekennzeichnet, daß** man ein Harz-Linker-Addukt der Formel (IV)

$$[\text{ Harzpolymer }] - [\text{ Linker - Z - } E^1 \text{ - } P(H)(=O) \text{ - O - } A^4]_n \quad (IV)$$

worin $A^4$, [Harzpolymer], Linker, Z, $E^1$ und n wie in Formel (VII) definiert sind, mit einem Elektrophil unter Ausbildung einer Phosphor-Kohlenstoffbindung zur Verbindungen der allgemeinen Formeln (VII) umsetzt.

**9.** Verfahren zur Herstellung von Verbindungen der Formel (I),

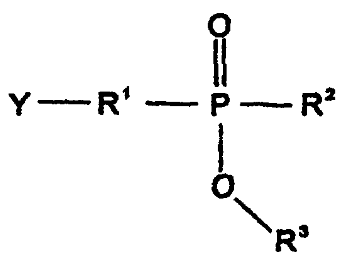

(I)

**dadurch gekennzeichnet, daß** man die Verbindung der Formel (I) aus einem Harz-Linker-Addukt der Formel (IX)

$$[\text{ Harzpolymer }] - [\text{ Linker - Z - } R^1 \text{ - } P(R^2)(=O) \text{ - O - } R^3]_n \quad (IX)$$

abspaltet, wobei in den Formeln (I) und (IX) die Reste [Harzpolymer], Linker, Z, Y, $R^1$, $R^2$, $R^3$ und die Zahl n wie in Anspruch 1 definiert sind.

**10.** Verbindungen der Formeln (II), (IV), (IV)', (V), (V)', (VI), (VI)', (VII), (VII)', (VIII), (VIII)' und (IX), wie in einem der Ansprüche 1 bis 9 definiert sind.

**11.** Substanzbibliothek enthaltend Verbindungen der Formel (II), (IV), (IV)', (V), (V)', (VI), (VI)', (VII), (VII)', (VIII), (VIII) ' oder (IX), wie gemäß einem der Ansprüche 1 bis 9 definiert sind, oder deren Gemische.

**12.** Substanzbibliothek enthaltend Verbindungen der Formel (I), wie sie in Anspruch 1 definiert sind, **dadurch gekennzeichnet, daß** die Substanzbibliothek unter Verwendung eines Verfahrens gemäß einem der Ansprüche 1 bis 9 hergestellt worden ist.

**13.** Verfahren zur Herstellung einer Substanzbibliothek enthaltend Verbindungen der Formel (I), (II), (IV), (IV)', (V), (V)', (VI), (VI)', (VII), (VII)', (VIII'), (VIII)' oder (IX) oder deren Gemische, **dadurch gekennzeichnet, daß** man die Verbindungen nach einem der Ansprüche 1 bis 9 herstellt und diese Verbindungen oder deren Gemische strukturbezogen anordnet.

**14.** Verwendung einer Substanzbibliothek, wie sie nach Anspruch 11, 12 oder 13 definiert ist, in einem Testverfahren auf biologische Wirkung als Pharmazeutikum oder Pflanzenschutzmittel.

## Claims

**1.** A process using intermediates which are linked to a resin polymer for preparing chemical compounds of the formula (I)

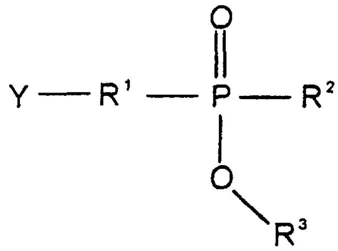 (I)

in which

- $R^1$ is an unsubstituted or substituted aromatic or heteroaromatic radical,
- $R^2$ is hydrogen or an organic radical which may also be linked to the rest of the compound of the formula (I) via hetero atoms,
- $R^3$ is hydrogen or an organic radical which is attached via a carbon atom and
- Y is the functional group which is formed at the molecule of the formula (I) after the compound (I) has been cleaved off from the resin polymer,

which comprises

a) reacting a resin-linker adduct of the formula (II)

$$\text{[resin polymer] - [linker-Z-E}^1\text{-S}^1]_n \qquad \text{(II)}$$

in which

| | |
|---|---|
| [resin polymer] | is the radical of a resin which, in the resin-linker compound (II), is connected via n binding sites with the n groups of the formula [linker-Z-$E^1$-$S^1$], |
| linker | is in each case an organic linker, |
| Z | is a linker-specific functional group or bond which, after cleavage of the compound (I) from the resin polymer-linker radical, gives rise to the group Y in formula (I), |
| $E^1$ | is defined as $R^1$ in formula (I) or is a radical which is suitable for preparing $R^1$ in compound (I), |
| $S^1$ | is a functional group suitable for palladium-catalyzed substitutions analogous to the Heck reaction, |
| n | is the number of the functional groups [linker-Z-$E^1$-$S^1$] at the resin, which depends on the molecular weight of the resin polymer and is greater than or equal to 1, |

in the presence of a suitable palladium catalyst with a compound selected from the group of the phosphinates (derivatives of hypophosphoric acid) of the formula (III)

$$A^1\text{-O-(PHO)}A^* \qquad \text{(III)}$$

in which

- $A^1$ is hydrogen or an organic radical,
- $A^*$ is a group which can be removed hydrolytically or after an intermediate reaction,

with substitution of the group $S^1$ to give a resin-bound compound of the formula (IV)

$$[\text{resin polymer}] - [\text{linker-Z-E}^1\text{-P(H) (=O)-O-A}^1\ ]_n \qquad (IV)$$

in which $A^1$ is as defined in formula (III), and

b) derivatizing, if appropriate, the compound (IV) in one or more further reaction steps at the organic radical $E^1$ to give the radical $(E^1)'$, thus yielding one or more resin-bound intermediates of the formula (IV)'

$$[\text{resin polymer}]\text{-}[\text{linker-Z-(E}^1)'\text{-P(H) (=O)-O-A}^1]_n \qquad (IV)'$$

in which $A^1$ is as defined in formula (III), and

c) hydrolyzing, if appropriate, the compound of the formula (IV) or (IV)' from step a) or b) to give a compound (V) or (V)' suitable for the resin-bound synthesis

$$[\text{resin polymer}] - [\ \text{linker-Z-E}^1\text{-P(H) (=O)-OH}]_n \qquad (V)$$

$$[\text{resin polymer}] - [\text{linker-Z-(E}^1)'\text{-P(H) (=O)-OH}]_n \qquad (V)'$$

and

d) esterifying, if appropriate, the compound (V) or (V)' obtained according to c) to give the compound of the formula (VI) or (VI)'

$$[\text{resin polymer}] - [\text{linker-Z-E}^1\text{-P(H) (=O)-O-R}^3]_n \qquad (VI)$$

$$[\text{resin polymer}] - [\text{linker-Z-(E}^1)'\text{-P(H)(=O)-O-R}^3]_n \qquad (VI)'$$

in which

$R^3$ is defined as $R^3$ in formula (I), but is not hydrogen, and

e) reacting, if appropriate, a compound (IV), (V) or (VI) or (IV)', (V)' or (VI)' obtained according to a), b), c) or d), whose common structural feature is the phosphonous acid or phosphonous ester group, forming a phosphorus-carbon bond, to give compounds of the formulae (VII) or (VIII) or (VII)' or (VIII)'

$$[\text{resin polymer}] - [\text{linker-Z-E}^1\text{-P(R}^2\text{) (=O) -O-A}^4]_n \qquad (VII)$$

$$[\text{resin polymer}] - [\text{linker-Z-(E}^1)'\text{-P(R}^2\text{) (=O)-O-A}^4]_n \qquad (VII)'$$

$$[\text{resin polymer}] - [\text{linker-Z-E}^1\text{-P(E}^2\text{) (=O)-O-A}^4]_n \qquad (VIII)$$

$$[\text{resin polymer}]\text{-}[\text{linker-Z-(E}^1)'\text{-P(E}^2\text{) (=O)-O-A}^4]_n \qquad (VIII)'$$

in which

$R^2$ is as defined in formula (I),
$E^2$ is an organic radical which can be derivatized to give the radical $R^2$,

$A^4$ = $A^1$, H or $R^3$, and

f) modifying the compounds obtained according to the abovementioned steps if required at the radicals $E^1$, $(E^1)'$, $E^2$ and $A^4$ in such a manner that the resin-bound compound of the formula (IX) is obtained

$$[\text{resin polymer}]\text{-}[\text{linker-Z-R}^1\text{-P(R}^2\text{) (=O)-O-R}^3]_n \qquad (IX)$$

in which $R^1$, $R^2$, $R^3$ are as defined in formula (I), and

g) cleaving the compound of the formula (I) from the resin-linker adduct of the formula (IX), where in the formulae (IV) to (IX) and (IV)' to (VIII)' the radicals [resin polymer], linker, Z are as defined in formula (II) and $E^1$ or $(E^1)'$ in the formulae (V) to (VIII) or (V)' to (VIII)' are as defined in formula (IV) or (IV)'.

**2.** The process as claimed in claim 1, wherein

$R^1$ is phenylene which is unsubstituted or substituted by 1 to 4 radicals selected from the group consisting of halogen, alkyl, haloalkyl, alkoxy, haloalkoxy, alkylthio, hydroxyl, amino, nitro, cyano, azido, alkoxycarbonyl, alkylcarbonyl, formyl, carbamoyl, mono- and dialkylaminocarbonyl, acylamino, mono- and dialkylamino, alkylsulfinyl, haloalkylsulfinyl, alkylsulfonyl and haloalkylsulfonyl, where each substituent may have up to 6 carbon atoms in the alkyl moiety, or is a heteroaromatic radical selected from the group consisting of the 5- or 6-membered ring having in each case 1, 2 or 3 hetero atoms selected from the group consisting of N, O and S, where the radical is unsubstituted or substituted by 1 to 4 radicals selected from the group consisting of halogen, alkyl, haloalkyl, alkoxy, haloalkoxy, alkylthio, hydroxyl, amino, nitro, cyano, azido, alkoxycarbonyl, alkylcarbonyl, formyl, carbamoyl, mono- and dialkylaminocarbonyl, substituted amino such as acylamino, mono-and dialkylamino, alkylsulfinyl, haloalkylsulfinyl, alkylsulfonyl and haloalkylsulfonyl, and where each substituent may have up to 6 carbon atoms in the alkyl moiety, and

$R^2$ is hydrogen, an aliphatic hydrocarbon radical which is unsubstituted or substituted and contains, inclusive of substituents, 1 to 30 carbon atoms,

$R^3$ is hydrogen or an aliphatic hydrocarbon radical which is unsubstituted or substituted and contains, inclusive of substituents, 1 to 30 carbon atoms, or is an aryl or heteroaryl radical which is unsubstituted or substituted and contains, inclusive of substituents, 1 to 30 carbon atoms, and

Y is H, COOH, $CONH_2$, OH, $NH_2$ or alkylamino.

**3.** The process as claimed in claim 1 or 2, wherein

$R^2$ is hydrogen or an aliphatic acyclic or cyclic hydrocarbon radical having 1 to 20 carbon atoms or heterocyclyl having 3 to 7 ring atoms and 1, 2 or 3 hetero atoms selected from the group consisting of N, O and S, where the hydrocarbon radical or the heterocyclyl radical is in each case unsubstituted or substituted by one or more radicals selected from the group consisting of halogen, alkoxy, alkenyloxy, alkynyloxy, haloalkoxy, haloalkenyloxy, haloalkynyloxy, alkylthio, amino, nitro, cyano, azido, alkoxycarbonyl, alkenyloxycarbonyl, alkynyloxycarbonyl, alkylcarbonyl, alkenylcarbonyl, alkynylcarbonyl, formyl, carbamoyl, mono- and dialkylaminocarbonyl, acylamino, mono- and dialkylamino, alkylsulfinyl, haloalkylsulfinyl, alkylsulfonyl, haloalkylsulfonyl, unsubstituted and substituted cycloalkyl, unsubstituted and substituted cycloalkenyl, unsubstituted and substituted aryl, unsubstituted and substituted heterocyclyl, unsubstituted and substituted cycloalkoxy, unsubstituted and substituted cycloalkenyloxy, unsubstituted and substituted aryloxy, unsubstituted and substituted heterocyclyloxy, unsubstituted and substituted cycloalkylamino, unsubstituted and substituted cycloalkenylamino, unsubstituted and substituted arylamino, unsubstituted and substituted heterocyclylamino, and in the case of cyclic radicals also alkyl and haloalkyl.

**4.** The process as claimed in claim 3, wherein

$R^2$ is a radical of the formula ($R^2$a), ($R^2$b), ($R^2$c), ($R^2$d) or ($R^2$e),

$$\text{-CHOH-R*} \qquad (R^2a)$$

$$- CO-NH-R^* \qquad (R^2b)$$

$$- CHR^{**}-NH-R^* \qquad (R^2c)$$

$$-CR^aR^b-CR^cR^d-X-R^e \qquad (R^2d)$$

$$-R^* \qquad (R^2e)$$

in which

X is the electronegative functional group of a Michael acceptor,

| | |
|---|---|
| $R^*$ | is an aliphatic acyclic or cyclic hydrocarbon radical having 1 to 12 carbon atoms or heterocyclyl having 3 to 6 ring atoms and 1, 2 or 3 hetero atoms selected from the group consisting of N, 0 and S, where the hydrocarbon radical or the heterocyclyl radical is in each case unsubstituted or substituted by one or more radicals selected from the group consisting of halogen, alkoxy, alkenyloxy, alkynyloxy, haloalkoxy, haloalkenyloxy, haloalkynyloxy, alkylthio, amino, nitro, cyano, azido, alkoxycarbonyl, alkenyloxycarbonyl, alkynyloxycarbonyl, alkylcarbonyl, alkenylcarbonyl, alkynylcarbonyl, formyl, carbamoyl, mono- and dialkylaminocarbonyl, acylamino, mono- and dialkylamino, alkylsulfinyl, haloalkylsulfinyl, alkylsulfonyl, haloalkylsulfonyl, unsubstituted and substituted cycloalkyl, unsubstituted and substituted cycloalkenyl, unsubstituted and substituted aryl, unsubstituted and substituted heterocyclyl, unsubstituted and substituted cycloalkoxy, unsubstituted and substituted cycloalkenyloxy, unsubstituted and substituted aryloxy, unsubstituted and substituted heterocyclyloxy, unsubstituted and substituted cycloalkylamino, unsubstituted and substituted cycloalkenylamino, unsubstituted and substituted arylamino, unsubstituted and substituted heterocyclylamino, and in the case of cyclic radicals also alkyl and haloalkyl, |
| $R^{**}$ | is a radical selected from the group of the radicals defined for $R^*$ or |
| $R^*$ and $R^{**}$ | together are an alkylene bridge which is unsubstituted or substituted by one or more radicals which are, independently of one another, selected from the group of the substituents at the hydrocarbon radical for $R^*$, and |
| $R^a$, $R^b$, $R^c$, $R^d$, $R^e$ | independently of one another are in each case a radical selected from the group of the radicals defined for $R^*$ or |
| $R^a$, $R^c$ or $R^d$, $R^e$ or $R^c$, $R^e$ | in pairs are an alkylene bridge which is unsubstituted or substituted by one or more radicals which are, independently of one another, selected from the group of the substituents at the hydrocarbon radical for $R^*$. |

**5.** The process as claimed in any of claims 1 to 4, wherein

$$R^3 = (C_1\text{-}C_4)\text{alkyl and } Y = COOH.$$

**6.** The process as claimed in any of claims 1 to 5, wherein

Z is a group of the formula -O-CO- or -NH-CO- and

$S^1$ is an iodine atom.

**7.** A process for preparing a compound of the formula (IV)

$$[\text{resin polymer}] - [\text{linker-Z-}E^1\text{-P(H) (=O)-O-}A^1]_n \qquad (IV)$$

in which $A^1$, [resin polymer], linker, Z, $E^1$ and n are as defined in claim 1 and which comprises reacting a resin-linker adduct of the formula (II)

$$[\text{resin polymer}]\text{-}[\text{linker-Z-}E^1\text{-}S^1]_n \qquad (II)$$

in which
[resin polymer], linker, Z, $E^1$ and n are as defined in formula (IV) and
$S^1$ is a functional group suitable for palladium-catalyzed substitutions analogous to the Heck reaction,
in the presence of a suitable palladium catalyst with a compound selected from the group of the phosphinates (derivatives of the hypophosphoric acid) of the formula (III)

$$A^1\text{-O-(PHO)}A^* \qquad (III)$$

in which

$A^1$ is hydrogen or an organic radical,
$A^*$ is a group which can be removed hydrolytically or after an intermediate reaction,

with substitution of the group $S^1$ to give the compound of the formula (IV).

**8.** A process for preparing a compound of the formula (VII)

$$[\text{resin polymer}] - [\text{linker-Z-}E^1\text{-P(}E^2\text{) (=O)-O-}A^4]_n \qquad (VII)$$

in which $A^4$, [resin polymer], linker, Z, $E^1$, $E^2$ and n are as defined in claim 1, which comprises reacting a resin-linker adduct of the formula (IV)

$$[\text{resin polymer}]\text{-}[\text{linker-Z-}E^1\text{-P(H) (=O)-O-}A^4]_n \qquad (IV)$$

in which $A^4$, [resin polymer], linker, Z, $E^1$ and n are as defined in formula (VII), with an electrophile, forming a phosphorus-carbon bond, to give compounds of the formulae (VII).

**9.** A process for preparing compounds of the formula (I)

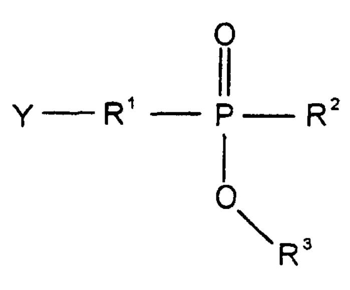
(I)

which comprises cleaving the compound of the formula (I) from a resin-linker adduct of the formula (IX)

$$[\text{resin polymer}] - [\text{linker-Z-}R^1\text{-P (}R^2\text{) (=O)-O-}R^3]_n \qquad (IX)$$

where in the formulae (I) and (IX) the radicals [resin polymer], linker, Z, Y, $R^1$, $R^2$, $R^3$ and the number n are as defined in claim 1.

**10.** A compound of the formula (II), (IV), (IV)', (V), (V)', (VI), (VI)', (VII), (VII)', (VIII), (VIII)' and (IX), as defined in any of claims 1 to 9.

**11.** A compound library, comprising compounds of the formula (II), (IV), (IV)', (V), (V)', (VI), (VI)', (VII), (VII)', (VIII), (VIII)' or (IX), as defined in any of claims 1 to 9, or mixtures thereof.

**12.** A compound library, comprising compounds of the formula (I) as defined in claim 1, which has been prepared using a process as claimed in any of claims 1 to 9.

**13.** A process for praparing a compound library, comprising compounds of the formula (I), (II), (IV), (IV)', (V), (V)', (VI), (VI)', (VII), (VII)', (VIII), (VIII)' or (IX) or mixtures thereof, wherein the compounds as claimed in any of claims 1 to 9 are prepared and these compounds or mixtures thereof are arranged according to their structure.

**14.** The use of the compound library as defined in claim 11, 12 or 13 in a test method for biological activity as medicament or crop protection agent.

**Revendications**

**1.** Procédé utilisant des produits intermédiaires, qui sont liés à une résine, pour la préparation de composés chimiques de formule (I)

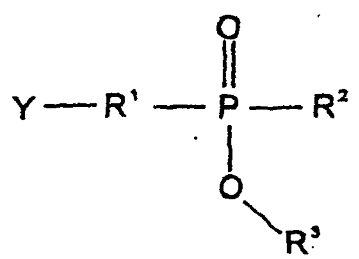

(I)

où

$R^1$ représente un reste aromatique ou hétéro-aromatique non substitué ou substitué,
$R^2$ représente un atome d'hydrogène ou un reste organique, qui peut également être lié par l'intermédiaire d'hétéroatomes à la partie restante du composé de formule (I),
$R^3$ représente un atome d'hydrogène ou un reste organique, qui peut également être lié par l'intermédiaire d'un atome de carbone et
Y représente le groupe fonctionnel, qui est formé sur la molécule de formule (I) après la séparation du composé (I) du polymère résineux,

**caractérisé en ce qu'**on fait réagir

a) un adduit de résine-lieur de formule (II)

$$\text{[polymère résineux] - [lieur-Z-}E^1\text{-}S^1]_n \qquad (II)$$

où
[polymère résineux] représente le reste d'une résine, qui est lié dans le composé résine-lieur (II) au moyen de n sites de liaison à n groupes de formule [lieur-Z-$E^1$-$S^1$],
lieur représente toujours un lieur organique,

Z représente une liaison ou un groupe fonctionnel spécifique de lieur respectif, à partir desquels est formé le groupe Y à la formule (I), après l'élimination du composé (I) du reste polymère résineux-lieur,

$E^1$ est défini comme $R^1$ à la formule (I) ou représente un reste approprié pour la préparation de $R^1$ dans le composé (I),

$S^1$ représente un groupe fonctionnel approprié pour les substitutions catalysées au palladium de façon analogue à la réaction de Heck,

n est le nombre des groupes fonctionnels [lieur-Z-$E^1$-$S^1$] sur le résine, qui dépend de la masse molaire et est supérieur ou égal à 1,

en présence d'un catalyseur au palladium approprié, sur un composé pris dans le groupe des phosphinates (dérivés de l'acide hypophosphoreux) de formule (III)

$$A^1\text{-O-(PHO)}A^* \qquad \text{(III)}$$

où

$A^1$ représente un atome d'hydrogène ou un reste organique,

$A^*$ représente un groupe séparable par hydrolyse ou après une réaction intermédiaire,

en substituant le groupe $S^1$, pour obtenir un composé de formule (IV) lié à la résine,

$$\text{[polymère résineux] - [lieur-Z-}E^1\text{-P(H) (=O) -O-}A^1]_n \qquad \text{(II)}$$

où $A^1$ est défini comme à la formule (III), et

b) éventuellement on transforme le composé (IV) sur le reste organique $E^1$ en reste $(E^1)'$ dans une ou plusieurs autres étapes réactionnelles et on obtient ainsi un ou plusieurs produits intermédiaires de formule (IV)' liés à la résine,

$$\text{[polymère résineux]-[lieur-Z-}(E^1)'\text{-P(H)(=O)-O-}A^1]_n \qquad \text{(IV)'}$$

où $A^1$ est défini comme à la formule (III), et

c) éventuellement on hydrolyse le composé de formule (IV), respectivement (IV)' de l'étape a) respectivement b) pour obtenir un composé de formule (V) respectivement (V)',

$$\text{[polymère résineux] - [lieur-Z-}E^1\text{-P(H) (=O)-OH]}_n \qquad \text{(V)}$$

$$\text{[polymère résineux] - [lieur-Z-}(E^1)'\text{-P(H) (=O)-OH]}_n \qquad \text{(V)'}$$

et

d) éventuellement on estérifie le composé (V) respectivement (V)' pour donner le composé de formule (VI) respectivement (VI)',

$$\text{[polymère résineux] - [lieur-Z-}E^1\text{-P(H) (=O)-O-}R^3]_n \qquad \text{(VI)}$$

$$\text{[polymère résineux] - [lieur-Z-}(E^1)'\text{-P(H)(=O)-O-}R^3]_n \qquad \text{(VI)'}$$

où

$R^3$ est défini comme $R^3$ à la formule (I), mais n'est pas égal à un atome d'hydrogène, et

e) éventuellement on fait réagir un composé de formules (IV), (V) ou (VI) respectivement (IV)', (V)' respectivement (VI)' dont la caractéristique structurale commune est le groupe acide phosphoneux ou le groupe ester de l'acide phosphoneux, en formant une liaison phosphore-carbone pour donner un composé de formules générales (VII) ou (VIII), ou (VII)' ou (VIII)',

$$[\text{polymère résineux}] - [\text{lieur-Z-E}^1\text{-P(R}^2\text{) (=O) -O-A}^4]_n \qquad \text{(VII)}$$

$$[\text{polymère résineux}] - [\text{lieur-Z-(E}^1\text{)'-P(R}^2\text{) (=O)-O-A}^4]_n \qquad \text{(VII)'}$$

$$[\text{polymère résineux}] - [\text{lieur-Z-E}^1\text{-P(E}^2\text{) (=O) -O-A}^4]_n \qquad \text{(VIII)}$$

$$[\text{polymère résineux}]- [\text{lieur-Z-(E}^1\text{)'-P(E}^2\text{) (=O)-O-A}^4]_n \qquad \text{(VIII)'}$$

où

$R^2$ possède la signification définie à la formule (I),
$E^2$ représente un reste organique qu'on peut transformer en reste $R^2$,
$A^4$ = $A^1$, H ou $R^3$, et

f) on modifie les composés obtenus selon les étapes précédentes, si nécessaire, sur les restes $E^1$, $(E^1)'$, $E^2$ et $A^4$ de façon telle qu'on obtienne le composé de formule (IX) lié à la résine,

$$[\text{polymère résineux}] - [\text{lieur-Z-R}^1\text{-P(R}^2\text{) (=O)-O-R}^3]_n \qquad \text{(IX)}$$

où $R^1$, $R^2$, $R^3$ sont définis comme à la formule (I), et
g) on sépare le composé de formule (I) de l'adduit résine-lieur de formule (IX),

aux formules (IV) à (IX) et (IV)' à (VIII)', les restes [polymère résineux], lieur, Z, étant définis comme à la formule (II) et $E^1$ ou $(E^1)'$ aux formules (V) à (VIII) respectivement (V)' à (VIII)' étant définis comme aux formules (IV) respetivement (IV)'.

**2.** Procédé selon la revendication 1,
**caractérisé en ce que**

$R^1$ représente un groupe phénylène, qui est non substitué ou substitué par un à quatre restes pris dans le groupe des restes halogène, alkyle, haloalkyle, alkoxy, haloalkoxy, alkylthio, hydroxy, amino, nitro, cyano, azido, alkoxycarbonyle, alkylcarbonyle, formyle, carbamoyle, mono- et dialkylaminocarbonyle, acylamino, mono-et dialkylamino, alkylsulfinyle, haloalkyl-sulfinyle, alkylsulfonyle et haloalkylsulfonyle, chaque substituant pouvant présenter jusqu'à 6 atomes de carbone dans le fragment alkyle, ou un reste hétéroaromatique pris dans le groupe des cycles de 5 à 6 chaînons présentant chacun 1, 2 ou 3 hétéroatomes pris dans le groupe des atomes de N, O et S, le reste étant non substitué ou substitué par un à quatre restes pris dans le groupe des restes halogène, alkyle, haloalkyle, alkoxy, haloalkoxy, alkylthio, hydroxy, amino, nitro, cyano, azido, alkoxycarbonyle, alkylcarbonyle, formyle, carbamoyle, mono- et dialkylaminocarbonyle, amino substitué, tel que acylamino, mono- et dialkylamino, alkylsulfinyle, haloalkylsulfinyle, alkylsulfonyle et haloalkylsulfonyle et chaque substituant pouvant présenter de préférence jusqu'à 6 atomes de carbone dans le fragment alkyle, et

$R^2$ représente un atome d'hydrogène, un reste hydrocarboné aliphatique, qui est non substitué ou substitué et présente, y compris les substituants, 1 à 30 atomes de carbone,
$R^3$ représente un atome d'hydrogène ou un reste hydrocarboné aliphatique, qui est non substitué ou substitué et présente, y compris les substituants, 1 à 30 atomes de carbone, ou un reste aryle ou hétéroaryle, qui est non substitué ou substitué et présente, y compris les substituants, 1 à 30 atomes

de carbone, et

Y représente H, COOH, $CONH_2$, OH, $NH_2$ ou alkylamino.

**3.** Procédé selon la revendication 1 ou 2, **caractérisé en ce que**

$R^2$ représente un atome d'hydrogène ou un reste hydrocarboné aliphatique acyclique ou cyclique ayant 1 à 20 atomes de carbone ou hétérocyclyle ayant 3 à 7 atomes nucléaires et 1, 2 ou 3 hétéroatomes pris dans le groupe des atomes de N, O et S, le reste hydrocarboné ou le reste hétérocyclyle étant chacun non substitué ou substitué par un ou plusieurs restes pris dans le groupe des restes halogène, alkoxy, alcényloxy, alcynyloxy, haloalkoxy, haloalcényloxy, haloalcynyloxy, alkylthio, amino, nitro, cyano, azido, alkoxycarbonyle, alcényloxycarbonyle, alcynyloxycarbonyle, alkylcarbonyle, alcénylcarbonyle, alcynylcarbonyle, formyle, carbamoyle, mono- et dialkylaminocarbonyle, acylamino, mono- et dialkylamino, alkylsulfinyle, haloalkylsulfinyle, alkylsulfonyle et haloalkylsulfonyle, cycloalkyle non substitué et substitué, cycloalcényle non substitué et substitué, aryle non substitué et substitué, hétérocyclyle non substitué et substitué, cycloalkoxy non substitué et substitué, cycloalcényloxy non substitué et substitué, aryloxy non substitué et substitué, hétérocyclyloxy non substitué et substitué, cycloalkylamino non substitué et substitué, cycloalcénylamino non substitué et substitué, arylamino non substitué et substitué, hétérocyclylamino non substitué et substitué, et dans le cas de restes cycliques, représente également alkyle et haloalkyle.

**4.** Procédé selon la revendication 3, **caractérisé en ce que**

$R^2$ représente un reste de formule ($R^2$a), ($R^2$b), ($R^2$c), ($R^2$d) ou ($R^2$e),

$$-CHOH-R^* \qquad (R^2a),$$

$$-CO-NH-R^* \qquad (R^2b),$$

$$-CHR^{**}-NH-R^* \qquad (R^2c),$$

$$-CR^aR^b-CR^cR^d-X-R^a \qquad (R^2d),$$

$$-R^* \qquad (R^2e),$$

où

X représente le groupe fonctionnel électronégatif d'un accepteur de Michael,

R* représente un reste hydrocarboné aliphatique, acyclique ou cyclique, présentant 1 à 12 atomes de carbone, ou hétérocyclyle présentant 3 à 6 atomes nucléaires et 1, 2 ou 3 hétéroatomes pris dans le groupe des atomes de N, O et S, le reste hydrocarboné ou le reste hétérocyclyle étant chacun non substitué ou substitué par un ou plusieurs restes pris dans le groupe des restes halogène, alkoxy, alcényloxy, alcynyloxy, haloalkoxy, haloalcényloxy, haloalcynyloxy, alkylthio, amino, nitro, cyano, azido, alkoxycarbonyle, alcényloxycarbonyle, alcynyloxycarbonyle, alkylcarbonyle, alcénylcarbonyle, alcynylcarbonyle, formyle, carbamoyle, mono- et dialkylaminocarbonyle, acylamino, mono- et dialkylamino, alkylsulfinyle, haloalkylsulfinyle, alkylsulfonyle et haloalkylsulfonyle, cycloalkyle non substitué et substitué, cycloalcényle non substitué et substitué, aryle non substitué et substitué, hétérocyclyle non substitué et substitué, cycloalkoxy non substitué et substitué, cycloalcényloxy non substitué et substitué, aryloxy non substitué et substitué, hétérocyclyloxy non substitué et substitué, cycloalkylamino non substitué et substitué, cycloalcénylamino non substitué et substitué, arylamino non substitué et substitué, hétérocyclylamino non substitué et substitué, et dans le cas de restes cycliques alkyle et haloalkyle également,

| | |
|---|---|
| $R^{**}$ | représente un reste pris dans le groupe des restes définis pour $R^*$ ou |
| $R^*$ et $R^{**}$ | représentent conjointement un pont alcényle, qui est non substitué ou substitué par un ou plusieurs restes, dont chacun est choisi indépendamment l'un de l'autre dans le groupe des substituants sur le reste hydrocarboné pour $R^*$, et |
| $R^a$, $R^b$, $R^c$, $R^d$, $R^e$ | représentent chacun, indépendamment les uns des autres, un reste pris dans le groupe des restes définis pour $R^*$ ou |
| $R^a$, $R^c$ ou $R^d$, $R^e$ ou $R^c$, $R^e$ | représentent par paires un pont alkylène qui est non substitué ou substitué par un ou plusieurs restes qui sont pris, indépendamment, dans le groupe des substituants sur le reste hydrocarboné pour $R^*$. |

5. Procédé selon l'une des revendications 1 à 4, **caractérisé en ce que** $R^3$ = alkyle en $C_1$-$C_4$ et Y = COOH.

6. Procédé selon l'une des revendications 1 à 5, **caractérisé en ce que**

Z représente un groupe de formule -O-CO- ou -NH-CO et
$S^1$ représente un atome d'iode.

7. Procédé pour la préparation d'un composé de formule (IV)

$$[\text{polymère résineux}] - [\text{lieur-Z-E}^1\text{-P(H) (=O) -O-A}^1]_n \quad \text{(IV)}$$

où $A^1$, [polymère résinex], lieur, E, $E^1$ et n sont définis comme à la revendication 1 et **caractérisé en ce qu'**on transforme un adduit résine-lieur de formule (II)

$$[\text{polymère résineux}] - [\text{lieur-Z-E}^1\text{-S}^1]_n \quad \text{(II)}$$

où
[polymère résineux], lieur, Z, $E^1$ et n sont définis comme à la formule (IV) et $S^1$ représente un groupe fonctionnel, qui est approprié aux substitutions catalysées au palladium analogues à la réaction de Heck,
en présence d'un catalyseur au palladium approprié, par un composé pris dans le groupe des phosphinates (dérivés de l'acide hypophosphoreux) de formule (III)

$$A^1\text{-O-(PHO)}A^* \quad \text{(III)}$$

où

$A^1$ représente un atome d'hydrogène ou un reste organique,
$A^*$ représente un groupe éliminable par hydrolyse ou après une réaction intermédiaire,

en substituant le groupe $S^1$, pour obtenir le composé de formule (IV).

8. Procédé pour la préparation d'un composé de formule (VII)

$$[\text{polymère résineux}] - [\text{lieur-Z-E}^1\text{-P(E}^2\text{) (=O)-O-A}^4]_n \quad \text{(VII)}$$

où $A^4$, [polymère résineux], lieur, Z, $E^1$, $E^2$ et n sont définis comme à la revendication 1, **caractérisé en ce qu'**on transforme un adduit résine-lieur de formule (IV)

$$[\text{polymère résineux}] - [\text{lieur-Z-E}^1\text{-P(H) (=O) -O-A}^4]_n \quad \text{(IV)}$$

où $A^4$, [polymère résineux], lieur, Z, $E^1$ et n sont définis comme à la revendication 1, par un électrophile en formant un composé phosphore-carbone pour obtenir des composés de formules générales (VII).

**9.** Procédé pour la préparation de cmposés de formule (I)

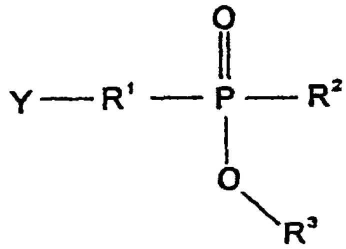

(1)

**caractérisé en ce qu'**on sépare le composé de formule

(I) d'un adduit résine-lieur de formule (IX)

$$[\text{polymère résineux}] - [\text{lieur-Z-R}^1\text{-P(R}^2\text{) (=O)-O-R}^3]_n \quad \text{(IX)}$$

dans les formules (I) et (IX), les restes [polymère résineux], lieur, Z, Y, $R^1$, $R^2$, $R^3$ et le nombre n sont définis à la revendication 1.

**10.** Composés de formules (II), (IV), (IV)', (V), (V)', (VI), (VI)', (VII), (VII)', (VIII), (VIII)' et (IX), étant définis à l'une des revendications 1 à 9.

**11.** Bibliothèque de substances contenant des composés de formules (II), (IV), (IV)', (V), (V)', (VI), (VI)', (VII), (VII)', (VIII), (VIII)' et (IX), étant définis selon l'une des revendications 1 à 9 ou leurs mélanges.

**12.** Bibliothèque de substances contenant des composés de formule (I) tels que définis à la revendication 1, **caractérisée en ce que** la bibliothèque de substances est constituée au moyen d'un procédé selon l'une des revendications 1 à 9.

**13.** Procédé pour la préparation d'une bibliothèque de substances contenant des composés de formules (I), (II), (IV), (IV)', (V), (V)', (VI), (VI)', (VII), (VII)', (VIII), (VIII)' et (IX) ou leurs mélanges, **caractérisée en ce qu'**on prépare les composés selon l'une des revendications 1 à 9 et on classe ces composés ou leurs mélanges en fonction de leur structure.

**14.** Utilisation d'une bibliothèque de substances telle que définie selon la revendication 11, 12 ou 13, dans un procédé d'essai de l'effet biologique en tant que produit pharmaceutique ou agent phytoprotecteur.